# EUROPEAN PATENT APPLICATION

(11) **EP 4 321 538 A1**
(43) Date of publication of application: **14.02.2024**
(21) Application number: 22775781.2
(22) Date of filing: 24.03.2022
(51) Int. Cl.: C07K 19/00, A61K 31/695, A61K 38/16, A61K 39/395, A61K 41/00, A61K 45/00, A61K 47/54, A61K 47/66, A61K 47/68, A61K 49/00, A61P 35/00, C07K 4/00, C07K 14/00, C07K 14/36, C07K 16/28, C12N 1/15, C12N 1/19, C12N 1/21, C12N 5/10, C12N 15/11, C12N 15/13

(54) **FUSION PROTEIN BETWEEN ANTIGEN-BINDING MOLECULE AND STREPTAVIDIN VARIANT**

(30) Priority: 25.03.2021 JP 2021051165; 02.09.2021 JP 2021142997
(71) Applicant: The University of Tokyo, Bunkyo-ku, Tokyo 113-8654 (JP); Savid Therapeutics Inc., Tokyo 168-0064 (JP)
(72) Inventor: TANAKA Toshiya, Tokyo 113-8654 (JP); KODAMA Tatsuhiko, Tokyo 113-8654 (JP); KANAI Motomu, Tokyo 113-8654 (JP); YAMATSUGU Kenzo, Tokyo 113-8654 (JP); TATSUMI Toshifumi, Tokyo 113-8654 (JP); TAKAHASHI Kazuki, Tokyo 113-8654 (JP); SUGIYAMA Akira, Tokyo 113-8654 (JP); TSUKAGOSHI Masanobu, Tokyo 168-0064 (JP)
(74) Representative: Godemeyer Blum Lenze Patentanwälte Partnerschaft mbB - werkpatent
(86) International application number: PCT/JP2022/013994
(87) International publication number: WO 2022/203000

(57) **Abstract**

It is an object of the present invention to provide a fusion protein of a molecule that recognizes cancer cells or the like and a mutant streptavidin, wherein the fusion protein is for use in the treatment or diagnosis of a cancer. According to the present invention, provided is a fusion protein, wherein an antigen-binding molecule having a molecular weight of 20,000 or less is bound, via a linker sequence, to the N-terminal side and/or C-terminal side of the amino acid sequence as set forth in SEQ ID NO: 1, provided that the C-terminal amino acid sequence consisting of Pro-Ser-Ala-Ala-Ser His-His-His-His-His-His may be partially or entirely deleted.

## Description

### Technical Field

The present invention relates to a fusion protein of an antigen-binding molecule and a mutant streptavidin and the use thereof.

### Background Art

Avidin and biotin, or streptavidin and biotin have an extremely high affinity between them (Kd = 10⁻¹⁵ to 10⁻¹⁴ M). This is one of the extremely strong interactions between two biomolecules. At present, the interaction between avidin/streptavidin and biotin has been widely applied in the field of biochemistry, molecular biology, or medicine. A drug delivery method and a pretargeting method have been devised, in which high binding ability between avidin/streptavidin and biotin is combined with an antibody molecule. In connection with these studies, a mutant streptavidin with a reduced affinity for natural biotin and a biotin-modified dimer having a high affinity for the mutant streptavidin with a low affinity for natural biotin are reported in Patent Document 1.

### Prior Art Documents

### Patent Documents

Patent Document 1: International Publication WO2015/125820

### Summary of Invention

### Object to be Solved by the Invention

It is an object of the present invention to provide a fusion protein of a molecule that recognizes cancer cells or the like and a mutant streptavidin, wherein the fusion protein is used to treat or diagnose cancer. It is another object of the present invention to provide a means for treating cancer or a means for diagnosing cancer, in which the above-described fusion protein is used.

### Means for Solving the Object

As a result of intensive studies directed towards achieving the above-described objects, the present inventor has selected a molecule having a molecular weight smaller than that of an antibody, as a molecule that recognizes cancer cells, and then, the present inventor has prepared a fusion protein of the aforementioned molecule and a mutant streptavidin. The present inventor has found that the proliferation of cancer cells can be suppressed by photoimmunotherapy using the above-described fusion protein and a conjugate of a biotin-modified dimer and a phthalocyanine dye, thereby completing the present invention.

Specifically, according to the present invention, the following inventions are provided.
<1> A fusion protein, wherein an antigen-binding molecule having a molecular weight of 20,000 or less is bound, via a linker sequence, to the N-terminal side and/or C-terminal side of the amino acid sequence as set forth in SEQ ID NO: 1, provided that the C-terminal amino acid sequence consisting of Pro-Ser-Ala-Ala-Ser His-His-His-His-His-His may be partially or entirely deleted.
<2> The fusion protein according to <1>, which has the antigen-binding molecule having a molecular weight of 20,000 or less, the linker sequence, and the amino acid sequence as set forth in SEQ ID NO: 1, provided that the C-terminal amino acid sequence consisting of Pro-Ser-Ala-Ala-Ser His-His-His-His-His-His may be partially or entirely deleted, in this order from the N-terminal side to the C-terminal side.
<3> The fusion protein according to <1> or <2>, wherein the antigen-binding molecule is a molecule that binds to an antigen expressing in a cancer cell.
<4> The fusion protein according to any one of <1> to <3>, wherein the antigen-binding molecule is a molecule that binds to Her2.
<5> The fusion protein according to any one of <1> to <4>, wherein the antigen-binding molecule has the amino acid sequence as set forth in SEQ ID NO: 2.
<6> The fusion protein according to any one of <1> to <5>, wherein the linker sequence consists of a glycine residue(s) and a serine residue(s) and the number of the amino acid residues is 5 to 25.
<7> The fusion protein according to any one of <1> to <6>, wherein the linker sequence is an amino acid sequence represented by [(Gly)ₘ-Ser]ₙ wherein m represents an integer of 1 to 10, and n represents an integer of 1 to 5.
<8> The fusion protein according to any one of <1> to <7>, which has the amino acid sequence as set forth in SEQ ID NO: 4, provided that the C-terminal amino acid sequence consisting of Pro-Ser-Ala-Ala-Ser His-His-His-His-His-His may be partially or entirely deleted.
<9> A nucleic acid encoding the fusion protein having the amino acid sequence as set forth in SEQ ID NO: 4, provided that the C-terminal amino acid sequence consisting of Pro-Ser-Ala-Ala-Ser His-His-His-His-His-His may be partially or entirely deleted.
<10> A cancer therapeutic agent or a cancer diagnostic agent, comprising the fusion protein according to any one of <1> to <8>.
<11> A kit for treating or diagnosing cancer, comprising (1) the fusion protein according to any one of <1> to <8>, and (2) a conjugate of a compound represented by the following formula (1) or a salt thereof, and a diagnostic substance or a therapeutic substance: 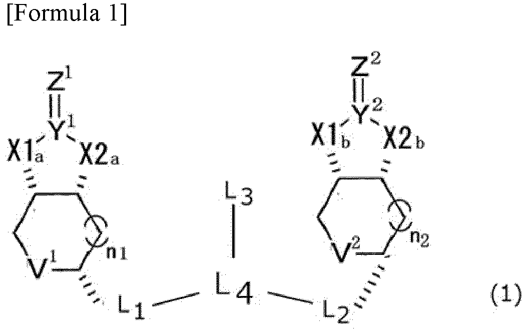 wherein
   X1a, X1b, X2a and X2b each independently represent O or NH,
   Y¹ and Y² each independently represent C or S,
   Z¹ and Z² each independently represent O, S or NH,
   V¹ and V² each independently represent S or S⁺-O⁻, n1 and n2 each independently represent an integer of 0 or 1,
   L₁ and L₂ each independently represent a divalent linking group,
   L₃ represents a group comprising a functional group capable of binding to the diagnostic substance or the therapeutic substance at the terminus thereof, and
   L₄ represents a trivalent linking group.
<12> The kit according to <11>, wherein the diagnostic substance or the therapeutic substance is a phthalocyanine dye.
<13> A method for producing the fusion protein according to any one of <1> to <8>, comprising a step of allowing a nucleic acid encoding the fusion protein according to any one of <1> to <8> to express in a host.
<14> The method according to <13>, wherein the fusion protein is allowed to express in a bacterial inclusion body and is then recovered.

### Advantageous Effects of Invention

By using the fusion protein of the present invention consisting of an antigen-binding molecule and a mutant streptavidin, for example, the proliferation of cancer cells can be suppressed.

### Brief Description of Drawings

[Fig. 1] Fig. 1 shows the results of tetramer purification according to a gel filtration method.
[Fig. 2] Fig. 2 shows the results of the measurement of affinity between FL and HER2.
[Fig. 3] Fig. 3 shows the results of the measurement of affinity between FL and Psyche.
[Fig. 4] Fig. 4 shows the results of a cytotoxicity assay using FL and the photosensitizer Psyche.
[Fig. 5] Fig. 5 shows the results of a mouse tumor growth inhibition assay using FL and the photosensitizer Psyche.
[Fig. 6] Fig. 6 shows the results of an *in vivo* experiment using FL and the photosensitizer Psyche (the treatment of subcutaneously transplanted tumor).
[Fig. 7] Fig. 7 shows the results of studies regarding the refolding of FL2-G5Sx3-del5.
[Fig. 8] Fig. 8 shows the results of confirmation of tetramer formation after refolding.
[Fig. 9] Fig. 9 shows the results of confirmation of cytotoxic activity by using the photosensitizer Psyche.
[Fig. 10] Fig. 10 shows an outline of the treatment of HER2-positive breast cancer by Z_{HER2:342}-Cupid-His-Ax-SiPC. Fig. 10A shows an outline of a pre-conjugate of Z_{HER2:342}-Cupid-His and Psyche-Ax-SiPC. The structural model of Z_{HER2:342}-Cupid-His was predicted by Alphafold2. Fig. 10B shows an experimental schedule using xenograft mouse models.

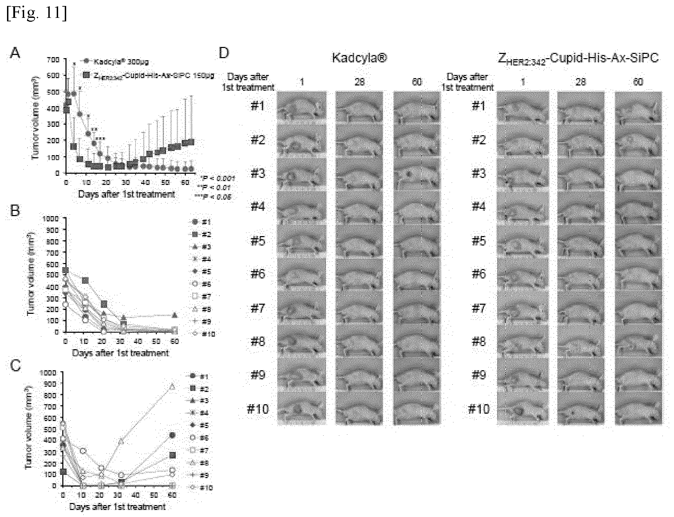

[Fig. 11] Fig. 11 shows the tumor volumes before and after a single-administration treatment. Fig. 11A shows the tumor growth curves of a Kadcyla group (300 µg/mouse, black circle) and a Z_{HER2:342}-Cupid-His-Ax-SiPC group (150 µg/mouse, black square). Fig. 11B shows the tumor growth curves of individual mice in the Kadcyla group (n = 10). Fig. 11C shows the tumor growth curves of individual mice in the Z_{HER2:342}-Cupid-His-Ax-SiPC group (n = 10). Fig. 11D shows representative animals having a tumor after single administration (left panels: Kadcyla group; and right panels: Z_{HER2:342}-Cupid-His-Ax-SiPC group).
[Fig. 12] Fig. 12 shows a second administration to a Z_{HER2:342}-Cupid-His-Ax-SiPC repetition group. Fig. 12A shows a tumor growth curve obtained after the 2nd treatment with the same dose (150 µg/mouse and the 2nd light irradiation) as in the 1st treatment. Fig. 12B shows the mice in the repetition group from the 1st day to the 32nd day after the 2nd treatment.
Fig. 12C shows the tumor volumes of individual mice the Kadcyla group (black circle) and in the Z_{HER2:342}-Cupid-His-Ax-SiPC group (black square) on the 97th day.
[Fig. 13] Fig. 13 shows the effects of tumor eradication by Kadcyla in histopathological examination. Histopathological analysis of skin and major organ tissues (liver, kidney, and lung) in xenograft mouse models at 97 days after the treatment with Kadcyla is shown. The black scale bar with the value (µm) in each picture indicates the size of a histological picture.
[Fig. 14] Fig. 14 shows the effects of tumor eradication by a Z_{HER2:342}-Cupid-His-Psyche-Ax-SiPC complex in histopathological examination. Histopathological analysis of skin and major organ tissues (liver, kidney, and lung) in xenograft mouse models at 97 days after the treatment with Z_{HER2:342}-Cupid-His-Psyche-Ax-SiPC is shown. The black scale bar with the value (µm) in each picture indicates the size of a histological picture.
[Fig. 15] Fig. 15 shows the time course of the individual weights in the Kadcyla group (A) and the Z_{HER2:342}-Cupid-His-Ax-SiPC group (B) after the first treatment. In Fig. 5B, the dotted line indicates the date of the second treatment with Z_{HER2:342}-Cupid-His-Ax-SiPC.

### Embodiment of Carrying out the Invention

Hereinafter, the present invention will be described in more detail.

### < Fusion protein of antigen-binding molecule and mutant streptavidin >

The fusion protein of the present invention is a fusion protein, in which an antigen-binding molecule having a molecular weight of 20,000 or less is bound, via a linker sequence, to the N-terminal side and/or C-terminal side of the amino acid sequence as set forth in SEQ ID NO: 1 (provided that the C-terminal amino acid sequence consisting of Pro-Ser-Ala-Ala-Ser His-His-His-His-His-His (or the C-terminal amino acid sequence consisting of 6 histidine residues) may be partially or entirely deleted). When the antigen-binding molecules each having a molecular weight of 20,000 or less is bound, via linker sequences, to both of the N-terminal side and/or C-terminal side of the amino acid sequence as set forth in SEQ ID NO: 1 (provided that the C-terminal amino acid sequence consisting of Pro-Ser-Ala-Ala-Ser His-His-His-His-His-His may be partially or entirely deleted), the antigen-binding molecules may be identical to or different from each other.

Preferably, the fusion protein of the present invention is a fusion protein having the antigen-binding molecule having a molecular weight of 20,000 or less, the linker sequence, and the amino acid sequence as set forth in SEQ ID NO: 1 (provided that the C-terminal amino acid sequence consisting of Pro-Ser-Ala-Ala-Ser His-His-His-His-His-His may be partially or entirely deleted) in this order from the N-terminal side to the C-terminal side.

The amino acid sequence as set forth in SEQ ID NO: 1 is the amino acid sequence of a mutant streptavidin, and specifically, this mutant streptavidin is the mutant streptavidin LISA314-V2122 described in Example 3 of International Publication WO2015/125820 (SEQ ID NO: 4 of International Publication WO2015/125820) (SEQ ID NO: 1 in the description of the present application).

As described above, the fusion protein of the present invention is a fusion protein of an antigen-binding molecule having a molecular weight of 20,000 or less and a mutant streptavidin. The fusion protein of the present invention forms a tetramer as a result of the affinity between the two amino acid sequences as set forth in SEQ ID NO: 1. When the protein having the amino acid sequence described in Example 2 is, for example, used as an antigen-binding molecule, the molecular weight of the tetramer formed by the fusion protein of the present invention is approximately 96 kDa. When the fusion protein as used in the present invention is administered to a living body to treat cancer, it is important to simultaneously achieve the following points: favorable tumor uptake; good clearance rate; and favorable tumor penetration. It is conceived that the above-described molecular weight (approximately 96 kDa) of the present invention is a molecular weight, with which the above-described three parameters can be simultaneously achieved.

It may be adequate if the molecular weight of the antigen-binding molecule is 20,000 or less. The molecular weight of the antigen-binding molecule is generally 4,000 or more and 20,000 or less, preferably 4,000 or more and 10,000 or less, and more preferably 4,000 or more and 8,000 or less.

The antigen-binding molecule of the present invention is a molecule having a concept different from an antibody. The molecular weight of an IgG antibody is generally approximately 150,000, whereas the antigen-binding molecule of the present invention is a molecule having a molecular weight much smaller than that of the IgG antibody. The antigen-binding molecule of the present invention may also be a molecule produced by imitating an antibody. For example, proteins each having a molecular weight of approximately 6,000 are produced by modifying a portion of the IgG-binding domain of Protein A (VDNKFNKEQQNAFYEILHLPNLNEEQRNAFIQSLKDDPSQSANLLAEAKKLNDAQ APK) (SEQ ID NO: 9), and the thus produced proteins are then screened, so that a desired antigen-binding molecule can be selected, as appropriate.

The antigen in the antigen-binding molecule is not particularly limited. The antigen is preferably an antigen that is expressed in cancer cells. Examples of the antigen that is specifically expressed in cancer may include the following antigens:

Epiregulin (EREG), ROBO 1, 2, 3 and 4, 1-40-β-amyloid, 4-1BB, 5AC, 5T4, ACVR2B, adenocarcinoma antigen, α-fetoprotein, angiopoetin 2, anthrax toxin, AOC3 (VAP-1), B-lymphoma cells, B7-H3, BAFF, β amyloid, C242 antigen, C5, CA-125, carbonic anhydrase 9 (CA-IX), cardiac myosin, CCL11 (eotaxin-1), CCR4, CCR5, CD11, CD18, CD125, CD140a, CD147 (basigin), CD147 (basigin), CD15, CD152, CD154 (CD40L), CD154, CD19, CD2, CD20, CD200, CD22, CD221, CD23 (IgE receptor), CD25 (IL-2 receptor α chain), CD28, CD3, CD30 (TNFRSF8), CD33, CD37, CD38 (cyclic ADP ribose hydrolase), CD4, CD40, CD41 (integrin α-IIb), CD44 v6, CD5, CD51, CD52, CD56, CD6, CD70, CD74, CD79B, CD80, CEA, CFD, ch4D5, CLDN18.2, *Clostridium difficile,* clumping factor A, CSF2, CTLA-4, cytomegalovirus, cytomegalovirus glycoprotein B, DLL4, DR5, *E. coli* Shiga toxin type *1, E. coli* Shiga toxin type 2, EGFL7, EGFR, endotoxin, EpCAM, episialin, ERBB3, *Escherichia coli,* F protein of respiratory syncytial virus, FAP, fibrin II β chain, fibronectin extra domain-B, folate receptor 1, Frizzled receptor, GD2, GD3 ganglioside, GMCSF receptor α chain, GPNMB, hepatitis B surface antigen, hepatitis B virus, HER1, HER2/neu, HER3, HGF, HIV-1, HLA-DRβ, HNGF, Hsp90, human β amyloid, human scatter factor receptor kinase, human TNF, ICAM-1 (CD54), IFN-α, IFN-γ, IgE, IgE Fc region, IGF-1 receptor, IGF-I, IgG4, IGHE, IL-1β, IL-12, IL-13, IL-17, IL-17A, IL-22, IL-23, IL-4, IL-5, IL-6, IL-6 receptor, IL-9, ILGF2, influenza A hemagglutinin, insulin-like growth factor I receptor, integrin α4, integrin α4β7, integrin α5β1, integrin α7β7, integrin αIIbβ3, integrin αvβ3, integrin γ induced protein, interferon receptor, interferon α/β receptor, ITGA2, ITGB2 (CD18), KIR2D, L-selectin (CD62L), Lewis-Y antigen, LFA-1 (CD11a), lipoteichoic acid, LOXL2, LTA, MCP-1, mesothelin, MS4A1, MUC1, mucin CanAg, myostatin, N-glycolylneuraminic acid, NARP-1, NCA-90 (granulocyte antigen), NGF, NOGO-A, NRP1, *Oryctolagus cuniculus,* OX-40, oxLDL, PCSK9, PD-1, PDCD1, PDGF-R α, phosphatidylserine, prostate cancer cells, *Pseudomonas aeruginosa,* Rabies virus glycoprotein, RANKL, respiratory syncytial virus, RHD, Rh (Rhesus) factor, RON, RTN4, sclerostin, SDC1, selectin P, SLAMF7, SOST, sphingosine-1-phosphate, TAG-72, TEM1, tenascin C, TFPI, TGFβ1, TGFβ2, TGF-β, TNF-α, TRAIL-R1, TRAII,-R2, tumor antigen CTAA16.88, MUC1 tumor-specific glycosylation, TWEAK receptor, TYRP1 (glycoprotein 75), VEGF-A, VEGFR-1, VEGFR2, vimentin, and VWF.

Among the above-described antigens, HER2 is particularly preferable.

An example of the antigen-binding molecule may be a protein having the amino acid sequence as set forth in SEQ ID NO: 2, which binds to HER2.

The linker sequence is not particularly limited, as long as the effects of the present invention can be achieved. The number of amino acids in the linker sequence is preferably 5 to 25 amino acids, more preferably 10 to 25 amino acids, and further preferably 15 to 20 amino acids.

A specific example of the linker sequence may be a sequence consisting of glycine residues and serine residues. As such a linker sequence, for example, an amino acid sequence represented by [(Gly)ₘ-Ser]ₙ (wherein m represents an integer of 1 to 10, and n represents an integer of 1 to 5) can be used. A specific example of the linker sequence may be Gly-Gly-Gly-Gly-Gly-Ser-Gly-Gly-Gly-Gly-Gly-Ser-Gly-Gly-Gly-Gly-Gly-Ser, but is not particularly limited thereto.

A specific example of the fusion protein of the present invention may be a fusion protein having the amino acid sequence as set forth in SEQ ID NO: 4 (provided that the C-terminal amino acid sequence consisting of Pro-Ser-Ala-Ala-Ser His-His-His-His-His-His may be partially or entirely deleted).

According to the present invention, a nucleic acid (for example, DNA) that encodes the above-described fusion protein of the present invention is further provided. A specific example of the nucleic acid of the present invention may be a nucleic acid that encodes the fusion protein having the amino acid sequence as set forth in SEQ ID NO: 4 (provided that the C-terminal amino acid sequence consisting of Pro-Ser-Ala-Ala-Ser His-His-His-His-His-His may be partially or entirely deleted). One example of the nucleic acid of the present invention may be a nucleic acid having the nucleotide sequence as set forth in SEQ ID NO: 3.

A nucleic acid (for example, DNA) encoding the fusion protein of the present invention can be used by being incorporated into a vector. In order to produce the fusion protein of the present invention, a nucleic acid encoding the fusion protein of the present invention is incorporated into an expression vector, and a host is then transformed with this expression vector, so that the fusion protein of the present invention can be expressed in the host. According to the present invention, there is a method for producing the fusion protein of the present invention, comprising a step of allowing a nucleic acid encoding the fusion protein of the present invention to express in a host. Preferably, the fusion protein can be expressed in a bacterial inclusion body and recovered therefrom.

When *Escherichia coli* is used as a host, the vector preferably has a replication origin (ori) and also has a gene for selecting the transformed host (e.g. a drug-resistance gene that is resistant to drugs, such as ampicillin, tetracycline, kanamycin or chloramphenicol, etc.). Moreover, an expression vector preferably has a promoter capable of efficiently expressing the mutant streptavidin of the present invention in a host, such as a lacZ promoter or a T7 promoter. Examples of such a vector include an M13 vector, a pUC vector, pBR322, pBluescript, pCR-Script, pGEX-SX-1 (Pharmacia), "QIAexpress system" (QIAGEN), pEGFP, and pET (in this case, BL21 that expresses T7 RNA polymerase is preferably used as a host).

A vector can be introduced into a host cell by applying a calcium chloride method or an electroporation method, for example. Further, a sequence that encodes a tag for improving solubility, such as glutathione S-transferase, thioredoxin or a maltose-binding protein, may be added. Still further, a sequence that encodes a tag designed for facilitating purification, such as a polyhistidine tag, a Myc epitope, a hemagglutinin (HA) epitope, a T7 epitope, an Xpress tag, a FLAG tag or other known tag sequences, may also be added.

Other than *Escherichia coli,* examples of the expression vector include: mammal-derived expression vectors (for example, pcDNA3 (manufactured by Invitrogen), pEGF-BOS (Nucleic Acids. Res. 1990, 18(17), p. 5322), pEF and pCDM8); insect cell-derived expression vectors (for example, "Bac-to-BAC baculovirus expression system" (manufactured by Gibco-BRL) and pBacPAK8); plant-derived expression vectors (for example, pMH1 and pMH2); animal virus-derived expression vectors (for example, pHSV, pMV and pAdexLcw); retrovirus-derived expression vectors (for example, pZIPneo); yeast-derived expression vectors (for example, "Pichia Expression Kit" (manufactured by Invitrogen), pNV11 and SP-Q01); and *Bacillus subtilis-derived* expression vectors (for example, pPL608 and pKTH50).

When the expression of the present fusion protein in an animal cell such as a CHO cell, a COS cell or an NIH3T3 cell is intended, it is essential for the expression vector to have a promoter necessary for the expression of the fusion protein in such an animal cell, such as an SV40 promoter (Mulligan et al., Nature (1979) 277, 108), an MMLV-LTR promoter, an EF1α promoter (Mizushima et al., Nucleic Acids Res. (1990) 18, 5322) or a CMV promoter. It is more preferable if the expression vector has a gene for selecting the transformation of a cell (for example, a drug-resistance gene capable of determining transformation with the use of drugs (neomycin, G418, etc.)). Examples of a vector having such properties include pMAM, pDR2, pBK-RSV, pBK-CMV, pOPRSV and pOP13.

The type of a host cell, into which the vector is introduced, is not particularly limited. Either prokaryotes or eukaryotes may be used. It is possible to use *Escherichia coli* or various types of animal cells, for example.

In the case of using a eukaryotic cell, for example, an animal cell, a plant cell or a fungal cell can be used as a host. Examples of an animal cell that can be used herein include: mammalian cells such as a CHO cell, a COS cell, a 3T3 cell, a HeLa cell or a Vero cell; and insect cells such as Sf9, Sf21 or Tn5. When the expression of a large amount of the fusion protein in an animal cell is intended, a CHO cell is particularly preferable. A vector can be introduced into a host cell by a calcium phosphate method, a DEAE-dextran method, a method using cationic ribosome DOTAP (manufactured by Boehringer Mannheim), an electroporation method, a lipofection method or the like.

As a plant cell, a cell from *Nicotiana tabacum* is known as a protein-producing system, for example. These cells may be subj ected to callus culture. Examples of a known fungal cell include: yeast cells including genus Saccharomyces such as *Saccharomyces cerevisiae;* and filamentous fungi including genus Aspergillus such as *Aspergillus niger.*

Examples of a prokaryotic cell that can be used herein include *Escherichia coli* (*E. coli*), such as JM109, DH5α or HB101. Moreover, *Bacillus subtilis* has been known.

These cells are transformed with the nucleic acid of the present invention, and the transformed cells are then cultured *in vitro,* so as to obtain the fusion protein of the present invention. The culture can be carried out in accordance with a known culture method. Examples of a culture solution of animal cells that can be used herein include DMEM, MEM, RPMI1640, and IMDM. During the culture, a serum infusion such as fetal calf serum (FCS) may be used in combination, or serum free culture may also be carried out. The pH applied during the culture is preferably approximately pH 6 to 8. The culture is generally carried out at a temperature of approximately 30°C to 40°C for approximately 15 to 200 hours. As necessary, medium replacement, ventilation and stirring are carried out. Furthermore, growth factors may also be added to promote the growth of cells.

### < Cancer therapeutic agent or cancer diagnostic agent >

The fusion protein of the present invention is useful as a cancer therapeutic agent or a cancer diagnostic agent.

According to the present invention, provided is a kit for treating or diagnosing cancer, comprising (1) the fusion protein of the present invention, and (2) a conjugate of a compound represented by a formula (1) as shown below or a salt thereof, and a diagnostic substance or a therapeutic substance.

When a molecule that is bound to an antigen existing in a cancer cell is used as such an antigen-binding molecule, the fusion protein of the present invention is administered to a patient, so that a mutant streptavidin can be accumulated specifically into cancer cells. Subsequently, by administering a conjugate of a biotin-modified dimer having an affinity for the mutant streptavidin and a diagnostic substance or a therapeutic substance to the patient, it becomes possible to accumulate the diagnostic substance or the therapeutic substance precisely into the cancer cells.

Otherwise, a complex is prepared by binding the "fusion protein of the present invention" with the "conjugate of a biotin-modified dimer having an affinity for a mutant streptavidin and a diagnostic substance or a therapeutic substance," and the thus prepared complex can be administered to the patient.

### < Biotin-modified dimer >

The biotin-modified dimer is a compound represented by the following formula (1) or a salt thereof, and is preferably a compound represented by the following formula (2) or a salt thereof. As such a biotin-modified dimer, the compound described in International Publication WO2015/125820 can be used. 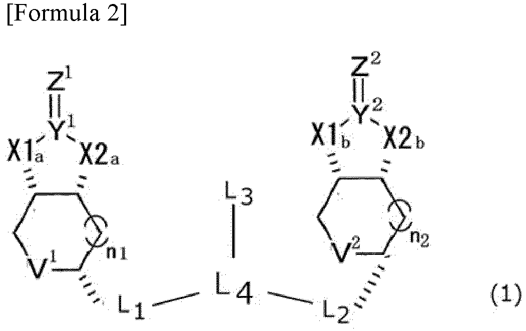 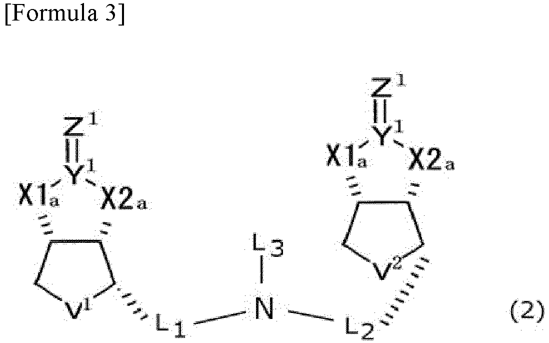 wherein
X1a, X1b, X2a and X2b each independently represent O or NH,
Y¹ and Y² each independently represent C or S,
Z¹ and Z² each independently represent O, S or NH,
V¹ and V² each independently represent S or S⁺-O⁻, n1 and n2 each independently represent an integer of 0 or 1,
L₁ and L₂ each independently represent a divalent linking group,
L₃ represents a group comprising a functional group capable of binding to the diagnostic substance or the therapeutic substance (for example, a phthalocyanine dye) at the terminus thereof, and
L₄ represents a trivalent linking group.

In the formula (1) and the formula (2), the portions represented by the following structures: 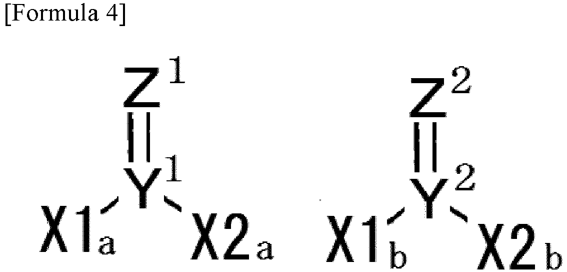 are preferably any one of the following portions, but are not limited thereto:

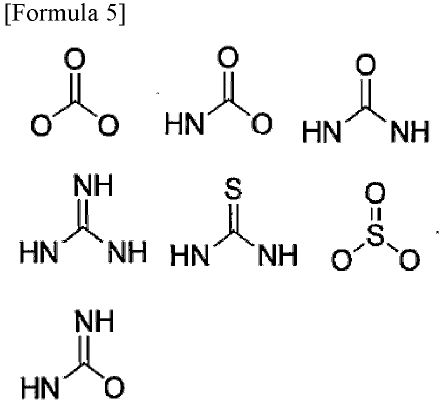

X1a, X1b, X2a and X2b preferably represent NH; Y¹ and Y² preferably represent C; Z¹ and Z² preferably represent NH; and V¹ and V² preferably represent S.

L₁ and L₂ each independently represent a divalent linking group consisting of a combination of groups selected from -CONH-, -NHCO-, -COO-, -OCO-, -CO-, -O-, and an alkylene group containing 1 to 10 carbon atoms.

Preferably, L₁ and L₂ each independently represent a divalent linking group consisting of a combination of groups selected from -CONH-, -NHCO-, -O-, and an alkylene group containing 1 to 10 carbon atoms.

Preferably, L₁ and L₂ each independently represent a divalent linking group consisting of a combination of groups selected from -CONH-, -NHCO-, and an alkylene group containing 1 to 10 carbon atoms.

L₄ represents a trivalent linking group, and is preferably the following: 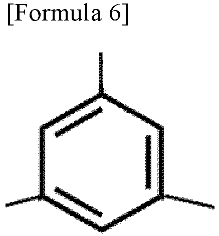 or, 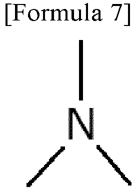 (which is a benzene-derived trivalent linking group or a nitrogen atom).

L₃ is preferably a group consisting of a combination of groups selected from - CONH-, -NHCO-, -COO-, -OCO-, -CO-, -O-, and an alkylene group containing 1 to 10 carbon atoms, and further comprising an amino group at the terminus thereof.

### < Conjugate of biotin-modified dimer and diagnostic substance or therapeutic substance >

By binding a diagnostic substance or a therapeutic substance to a biotin-modified dimer, a conjugate of the biotin-modified dimer and the diagnostic substance or the therapeutic substance can be prepared. Examples of the diagnostic substance or the therapeutic substance may include a fluorochrome, a chemiluminescent agent, a radioisotope, a sensitizer consisting of a metal compound or the like, a neutron-capturing agent consisting of a metal compound or the like, a phthalocyanine dye, a low-molecular-weight compound, micro- or nano-bubbles, and a protein. Preferably, a phthalocyanine dye can be used.

### < Phthalocyanine dye >

As a phthalocyanine dye, specifically, a compound represented by the following formula (1) or a salt thereof can be used.

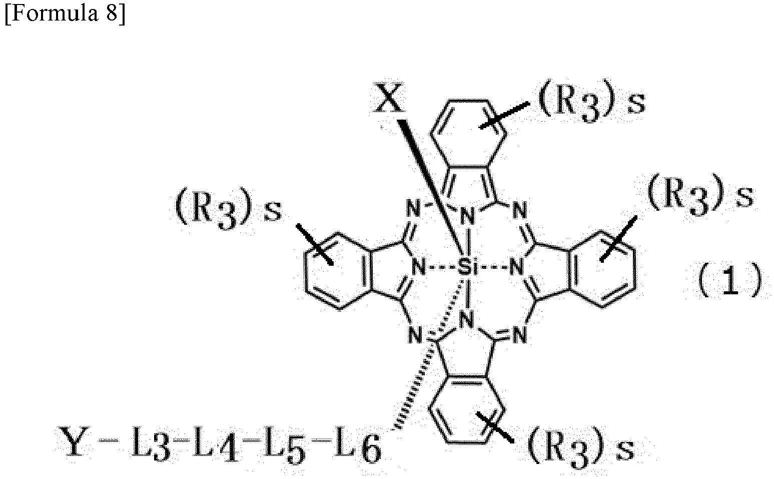

In the above formula (1), X represents a substituent having a hydrophilic group at the terminus thereof. The hydrophilic group is preferably a sulfonic acid group, a phosphoric acid group, an ammonium group, or the like.

X is preferably a substituent having a sulfonic acid group at the terminus thereof.

An example of X may be a group represented by the following formula:

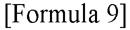

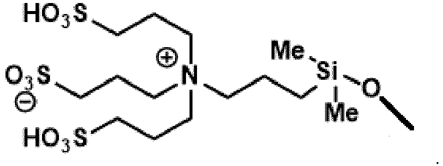

In the present description, Me represents a methyl group.

L₃, L₄, L₅, and L₆ each independently represent a divalent linking group.

L₃ is preferably the following: 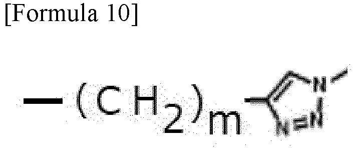 wherein m represents an integer of 1 to 5.

L₄ is preferably -[(CH₂)ₚ-O)]_{q}- (wherein p and q each independently represent an integer of 1 to 5).

L₅ is preferably -CONH-, -NHCO-, -COO-, or -OCO-, and is more preferably - CONH-.

L₆ is preferably -(CH₂)ᵣ-, -(CH₂)ᵣ-O-, or -(CH₂)ᵣ-Si(R¹)(R²)-O- (wherein r represents an integer of 1 to 5, and R¹ and R² each independently represent an alkyl group containing 1 to 4 carbon atoms). R¹ and R² are particularly preferably methyl groups.

Y represents a group capable of binding to an antigen-binding molecule. Y is preferably an active ester of a carboxyl group, and is more preferably an succinimidyl ester of a carboxyl group.

R₃ represents an alkyl group containing 1 to 6 carbon atoms, an alkoxy group containing 1 to 6 carbon atoms, a halogen atom, -SR₁₁, -SOR₁₂, an aryl group containing 6 to 10 carbon atoms, -N(R₁₃)(R₁₄), or -NO₂, or two adjacent R₃s may together form an aryl group containing 6 to 10 carbon atoms. R₁₁, R₁₂, R₁₃, and R₁₄ each independently represent an alkyl group containing 1 to 6 carbon atoms or an alkoxy group containing 1 to 6 carbon atoms.

Herein, the aryl group may be optionally substituted with an alkyl group containing 1 to 6 carbon atoms or an alkoxy group containing 1 to 6 carbon atoms.

Herein, the alkyl group and the alkoxy group may be optionally substituted with a halogen atom.

When a plurality of R₃s are present, the plurality of R₃s may be identical to or different from one another.

s represents an integer of 0 to 4. s is preferably 0.

The halogen atom may be any of fluorine, chlorine, bromine and iodine, and the halogen atom is preferably fluorine, chlorine, or bromine.

The compound of the phthalocyanine dye can be synthesized in accordance with the methods described in Production Examples 1 to 5 in the after-mentioned Examples.

### < Photoimmunotherapy >

Photoimmunotherapy is a therapeutic method of using a photosensitizer and an irradiation light to destroy specific cells in a body. When a photosensitizer is exposed to a light with a specific wavelength, it generates cytotoxic reactive oxygen species capable of inducing apoptosis, necrosis, and/or autophagy to around cells. For example, Japanese Patent No. 6127045 discloses a method of killing cells, comprising: a step of allowing cells comprising a cell surface protein to come into contact with a therapeutically effective amount of one or more antibodies-IR700 molecules, wherein the antibodies specifically bind to the cell surface protein; a step of irradiating the cells with a light at a wavelength of 660 to 740 nm and at a dose of at least 1 Jcm⁻²; and a step of allowing the cells to come into contact with one or more therapeutic agents at approximately 0 to 8 hours after the irradiation, thereby killing the cells. JP Patent Publication (Kohyo) No. 2017-524659 A discloses a method of inducing cytotoxicity to a subject affected with a disease or a pathology, comprising: (a) administering to a subject, a therapeutically effective drug comprising a phthalocyanine dye such as IRDye (registered trademark) 700DX conjugated with a probe specifically binding to the cell of the subject; and (b) irradiating the cell with an appropriate excitation light in an amount effective for inducing cell death.

The fusion protein of the present invention and the conjugate of a biotin-modified dimer and a phthalocyanine dye are administered to a subject, and the cells are then irradiated with an excitation light in an amount effective for suppression of cell proliferation or induction of cell death, so that the cell proliferation can be suppressed or the cell death can be induced, and thereby the subject can be treated.

Preferably, the fusion protein of the present invention and the conjugate of a biotin-modified dimer and a phthalocyanine dye are administered to a subject, and the cells are then irradiated with an excitation light in an amount effective for suppression of cell proliferation or induction of cell death, so that the cell proliferation can be suppressed or the cell death can be induced, and thereby the subject can be treated.

The subject used herein includes humans and non-human animals. Examples of the subject may include humans and experimental animals such as mice. The subject is preferably affected with a disease regarding which suppression of cell proliferation or induction of cell death is desired. For example, the subject is affected with a cancer or a solid tumor.

Examples of the "cancer" may include carcinoma, lymphoma, blastoma, sarcoma, and leukemia or malignant lymphoma. Specific examples of the cancer may include squamous cell carcinoma (e.g., epithelial squamous cell carcinoma), lung cancer including small cell lung cancer, non-small cell lung cancer ("NSCLC"), pulmonary adenocarcinoma and pulmonary squamous cell carcinoma, peritoneal cancer, hepatocarcinoma, corpus ventriculi or stomach cancer, including digestive cancer, pancreatic cancer, glioblastoma, cervical cancer, ovarian cancer, liver cancer, bladder cancer, hepatocellular cancer, breast cancer, colon cancer, rectal cancer, colorectal cancer, endometrial membrane cancer or endometrial carcinoma, salivary gland carcinoma, kidney or renal region cancer, prostate cancer, vulvar cancer, thyroid cancer, hepatocellular carcinoma, anal carcinoma, penile carcinoma, and head and neck cancer.

The solid tumor means a benign or malignant, abnormal cell mass that generally does not contain a capsule. Examples of the solid tumor may include glioma, astrocytoma, medulloblastoma, craniopharyngioma, ependymoma, pineal gland tumor, hemangioblastoma, acoustic neuroma, oligodendrocyte, meningioma, melanoma, neuroblastoma, and retinoblastoma.

Examples of the administration method to the subject may include, but are not limited to, a local route, an injection (a subcutaneous injection, an intramuscular injection, an intradermal injection, an intraperitoneal injection, an intratumoral injection, an intravenous injection, etc.), an oral route, an ocular route, a sublingual route, a rectal route, a percutaneous route, an intranasal route, a vaginal route, and an inhalation route.

It is preferable that the conjugate of a biotin-modified dimer and a phthalocyanine dye and the fusion protein of the present invention are each administered in a therapeutically effective amount. Regarding each of the above-described conjugate and fusion protein, the therapeutically effective amount per 60 kg is at least 0.5 mg (mg/60 kg), at least 5 mg/60 kg, at least 10 mg/60 kg, at least 20 mg/60 kg, at least 30 mg/60 kg, or at least 50 mg/60 kg. For example, when it is intravenously administered, the applied dose is 1 mg/60 kg, 2 mg/60 kg, 5 mg/60 kg, 20 mg/60 kg, or 50 mg/60 kg, and it is, for example, 0.5 to 50 mg/60 kg. In another example, the therapeutically effective amount is at least 100 µg/kg, at least 500 µg/kg or at least 500 µg/kg, and it is, for example, at least 10 µg/kg. For example, when it is intratumorally or intraperitoneally administered, the dose is 100 µg/kg, 250 µg/kg, approximately 500 µg/kg, 750 µg/kg, or 1000 µg/kg, and it is, for example, 10 µg/kg to 1000 µg/kg. In one example, when it is administered in the form of a solution for local administration, the therapeutically effective amount is 10 µg/ml, 20 µg/ml, 30 µg/ml, 40 µg/ml, 50 µg/ml, 60 µg/ml, 70 µg/ml, 80 µg/ml, 90 µg/ml, 100 µg/ml or the like, or it is 20 µg/ml to 100 µg/ml, or it is at least 500 µg/ml, or at least 1 µg/ml.

The above-described dose can be administered once or divided doses over several administrations (2, 3, or 4 times, etc.), or as a single preparation.

The conjugate of a biotin-modified dimer and a phthalocyanine dye and the fusion protein of the present invention can be each administered alone, or can also be administered in the presence of a pharmaceutically acceptable carrier, or can also be administered in the presence of other therapeutic agents (other anticancer agents, etc.).

The conjugate of a biotin-modified dimer and a phthalocyanine dye and the fusion protein of the present invention can bind to target cells or target tissues, such as circulating tumor cells or solid tumor cells. Thereafter, the target cells or tissues are irradiated with a light, so that the above-described conjugate or complex can absorb the light and can damage or destroy the target cells or tissues.

In the photoimmunotherapy, the wavelength of the irradiation light is preferably 660 to 740 nm, and the irradiation light has a wavelength of, for example, 660 nm, 670 nm, 680 nm, 690 nm, 700 nm, 710 nm, 720 nm, 730 nm, or 740 nm. Light irradiation may be carried out using a device equipped with a near infrared (NIR) light emitting diode.

The light irradiation amount is at least 1 J/cm², for example, at least 4 J/cm², at least 10 J/cm², at least 15 J/cm², at least 20 J/cm², at least 50 J/cm², or at least 100 J/cm². It is, for example, 1 to 500 J/cm². Light irradiation may be carried out several times (e.g., 2, 3, 4, 5, 6, 7, 8, 9, or 10 times).

The present invention will be more specifically described in the following examples. However, these examples are not intended to limit the scope of the present invention.

### Examples

### < Production Example 1: Synthesis of Compound 2 >

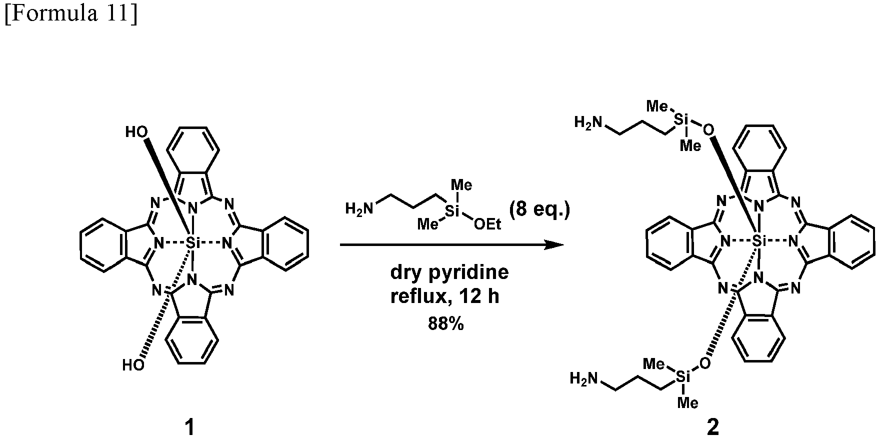

(3-Aminopropyl)dimethylethoxysilane (113 mg, 700 µmol) and dry pyridine (30 mL) were added to silicon phthalocyanine dihydroxide 1 (50 mg, 87 µmol), and the obtained mixture was stirred and heated to reflux for 5 hours. Thereafter, the solvent was removed under reduced pressure, and the residue was then purified by silica gel column chromatography (gradient: 1% for 3 min; 1-10% for 15min; 10-20% for 10 min CH₃OH in CH₂Cl₂, Yamazen Corporation Universal^{™} Column Amino 40 µm 60Å 2.3 x 12.3 cm, 16 g, flow rate = 10 mL/min) to obtain target Compound 2 (62 mg, 77 µmol, 88%, dark blue).
¹H NMR (500 MHz, CDCl₃): δ 9.65 (dd, *J* = 2.8 Hz, 5.7 Hz, 8H), 8.34 (dd, *J* = 2.8 Hz, 5.7 Hz, 8H), 1.18 (t, *J* = 7.6 Hz, 4H), -1.23 (m, 4H), -2.30 (m, 4H), -2.86 (s, 12H).
LRMS (ESI): *m*/*z* 805.30 [M+H] ⁺

### < Production Example 2: Synthesis of Compound 4 >

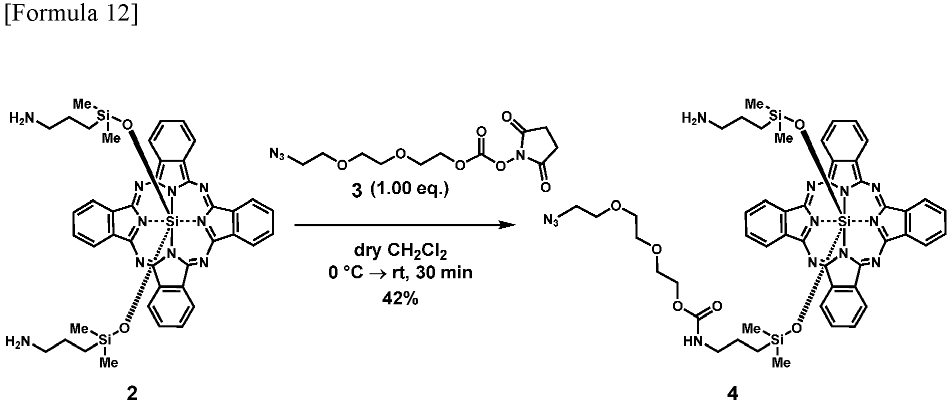

A dry dichloromethane (27 mL) solution, in which Compound 2 (100 mg, 124 µmol) was dissolved, was cooled to 0 °C. Thereafter, a dry dichloromethane (3 mL) solution of Compound **3** (39.2 mg, 124 µmol) was slowly added to the obtained solution, while stirring, and the obtained mixture was then stirred at room temperature for 30 minutes, while shielding the light with an aluminum foil. Thereafter, the solvent was removed under reduced pressure, and the residue was then purified by silica gel column chromatography (gradient: 1% for 3 min; 1-5% for 15 min CH₃OH in CH₂Cl₂, Yamazen Corporation Universal^{™} Column Amino 40 µm 60Å 2.3 x 12.3 cm, 16 g, flow rate = 10 mL/min) to obtain target Compound **4** (52.3 mg, 51.9 µmol, 42%, dark blue).
¹H NMR (500 MHz, CDCl₃): δ 9.65 (dd, *J* = 2.8 Hz, 5.7 Hz, 8H), 8.35 (d, *J* = 2.8 Hz, 5.7 Hz, 8H), 4.08 (t, *J* = 4.8 Hz, 2H), 3.73-3.60 (m, 8H), 3.39 (t, *J* = 5.7 Hz, 2H), 1.76 (m, 2H), 1.18 (t, *J* = 6.7 Hz, 2H), -1.23 (m, 4H), -2.30 (m, 4H), -2.86 (s, 12H).
LRMS (ESI): *m*/*z* 1006.85 [M+H] ⁺

### < Production Example 3: Synthesis of Compound 5 >

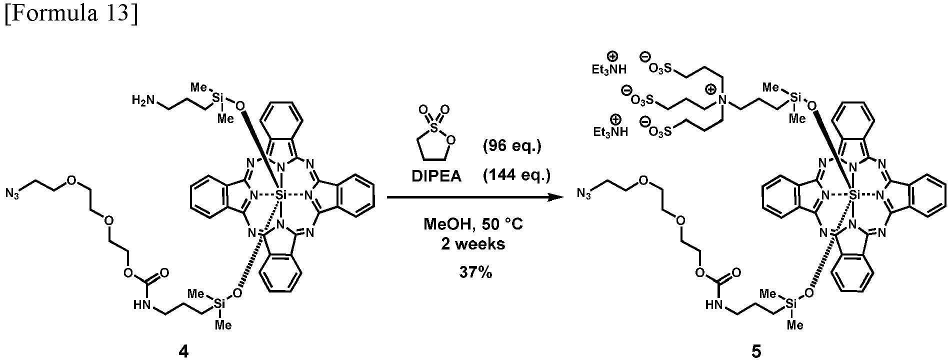

1,3-Propanesultone (86.2 mg, 707 µmol), diisopropylethylamine (DIPEA, 137 mg, 1.06 mmol), and methanol (3 mL) were added to Compound **4** (22.2 mg, 22.1 µmol), and the obtained mixture was then stirred at 50 °C, while shielding the light with an aluminum foil. On Days 7 and 10 after initiation of the reaction, 1,3-propanesultone (86.2 mg, 707 µmol) and DIPEA (137 mg, 1.06 mmol) were added to the reaction mixture. On Day 14 after initiation of the reaction, volatiles were removed under reduced pressure, and the residue diluted with water/acetonitrile (1 : 1) to 6000 µL was divided into two aliquots, which were then purified by reverse phase HPLC (gradient: 50% for 5 min; 50-80% for 25 min; 80-100% for 10 min CH₃CN in 50 mM triethylammonium acetate aqueous solution (pH 7.0), retention time = 15.8 min, YMC-Actus Triart C18, flow rate = 10 mL/min), so as to obtain target Compound 5 (12.7 mg, 8.09 µmol, 37%, dark blue).
¹H NMR (500 MHz, CD₃OD): δ 9.72 (dd, *J* = 2.8 Hz, 5.7 Hz, 8H), 8.46 (dd, *J* = 2.8 Hz, 5.7 Hz, 8H), 3.97 (t, *J* = 4.8 Hz, 2H), 3.63-3.58 (m, 8H), 3.32 (m, 2H), 2.80-2.70 (m, 12H), 1.99 (t, *J* = 6.7 Hz, 2H), 1.70 (m, 6H), 1.60 (t, *J* = 6.7 Hz, 2H), -1.07 (m, 2H), -1.14 (m, 2H), - 2.12 (m, 2H), -2.28 (m, 2H), -2.82 (s, 6H), -2.89 (s, 6H).
LRMS (ESI): *m*/*z* 1372.55 [M+H] ⁺

### < Production Example 4: Synthesis of Compound 6 >

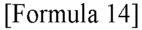

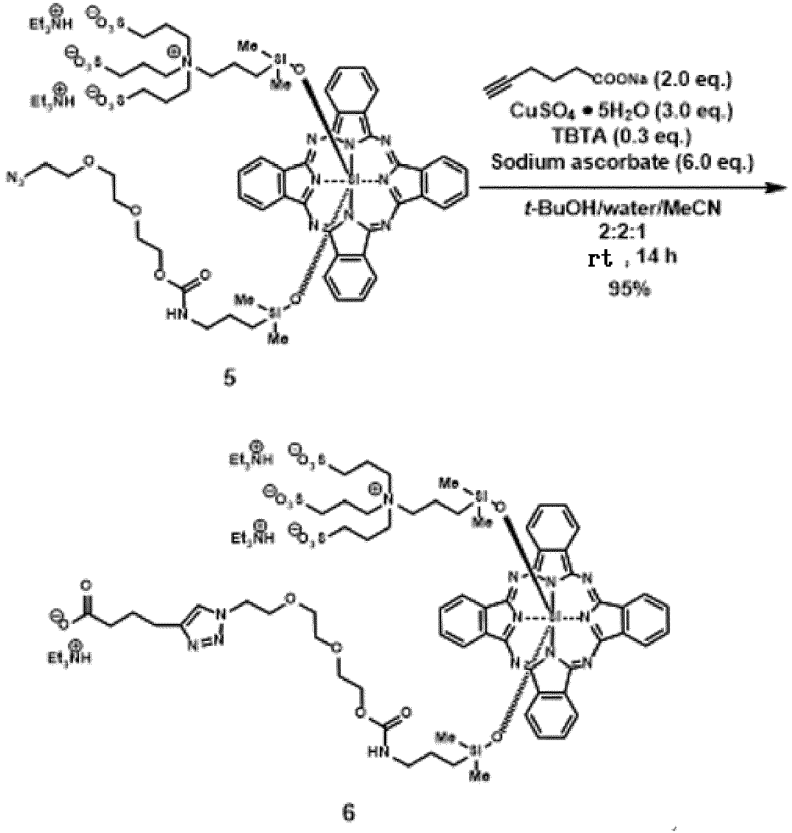

5-Hexynoic acid sodium salt (0.19 mg, 1.4 µmol), copper sulfate pentahydrate (0.71 mg, 2.8 µmol), tris[(1-benzyl-1*H*-1,2,3-triazol-4-yl)methyl]amine (0.15 mg, 0.28 µmol), L(+)-ascorbic acid sodium salt (1.1 mg, 5.7 µmol), *t*-butanol (300 µL), water (300 µL), and acetonitrile (150 µL) were added to Compound **5** (1.3 mg, 0.95 µmol), and the obtained mixture was then stirred at room temperature for 14 hours, while shielding the light with an aluminum foil. Thereafter, the solvent was removed under reduced pressure, and the residue diluted with water/acetonitrile (1 : 1) to 3000 µL was then purified by reverse phase HPLC (gradient: 30% for 5 min; 30-80% for 40 min; 80-100 for 10 min CH₃CN in 50 mM triethylammonium acetate aqueous solution (pH 7.0), retention time = 24.6 min, YMC-Triart C18, flow rate = 3.5 mL/min) to obtain target Compound **6** (1.6 mg, 0.90 µmol, 95 %, dark blue).
¹H NMR (500 MHz, CD₃OD): δ 9.73 (dd, *J* = 2.8 Hz, 5.7 Hz, 8H), 8.46 (dd, *J* = 2.8 Hz, 5.7 Hz, 8H), 7.75 (s, 1H), 4.48 (t, *J* = 4.8 Hz, 2H), 3.95 (t, *J* = 3.8 Hz, 2H), 3.83 (t, *J* = 4.8 Hz, 2H), 3.60-3.50 (m, 6H), 2.80-2.70 (m, 12H), 2.67 (t, J = 7.6 Hz, 2H), 2.24 (brt, 2H), 2.00 (t, *J* = 8.6 Hz, 2H), 1.90 (t, *J* = 7.6 Hz, 2H), 1.71 (quin, *J* = 7.6 Hz, 6H), 1.61 (t, *J* = 6.7 Hz, 2H), -1.03 (brt, 2H), -1.11 (brt, 2H), -2.13 (t, *J* = 8.6 Hz, 2H), -2.27 (t, *J* = 8.6 Hz, 2H), -2.80 (s, 6H), -2.88 (s, 6H).
LRMS (ESI): *m*/*z* 1485.15 [M+H] ⁺

### < Production Example 5: Synthesis of Compound 7 >

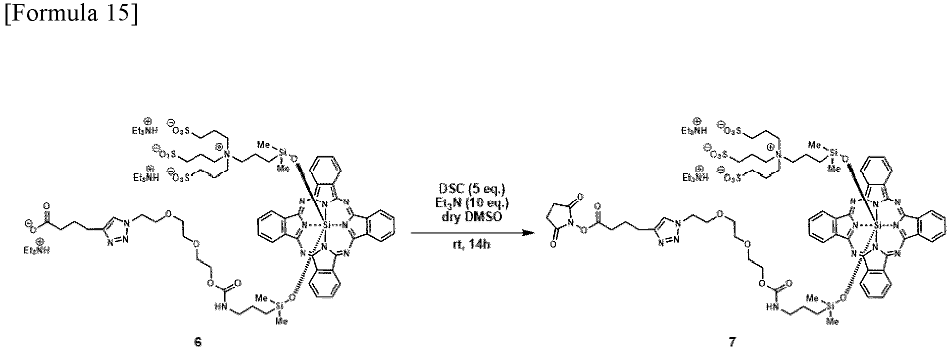

N,N'-Disuccinimidyl carbonate (DSC, 1.7 mg, 6.8 µmol), Et₃N (1.4 mg, 14 µmol), and dry dimethyl sulfoxide (500 µL) were added to Compound 6 (2.3 mg, 1.3 µmol), and the obtained mixture was then stirred at room temperature for 14 hours, while shielding the light with an aluminum foil. A precipitate was generated by adding diethyl ether (50 mL) to the reaction solution, and after the removal of a supernatant liquid, the precipitate was washed with diethyl ether (50 mL) to obtain Compound 7. This compound was directly used in the subsequent reaction without further purification.

### < Production Example 6: Synthesis of Compound 8 >

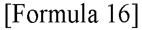

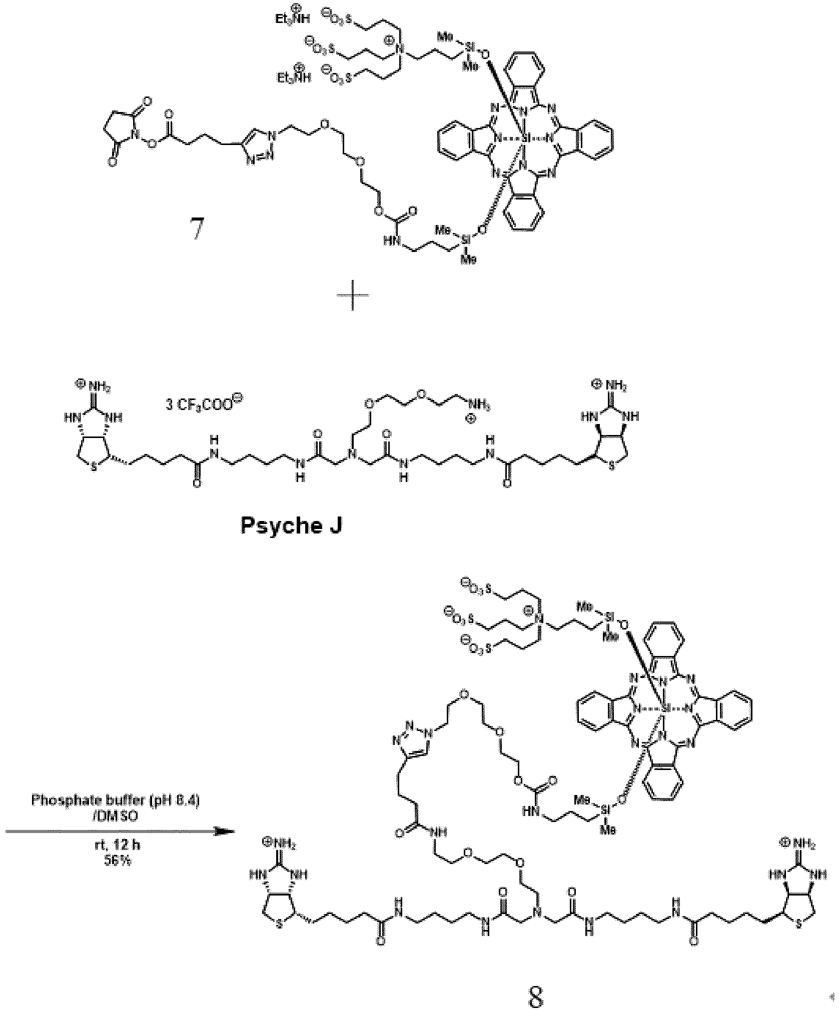

Compound 7 (2.3 mg, 1.3 µmol), a disodium hydrogen phosphate buffer (pH 8.4, 150 µL) and dimethyl sulfoxide (150 µL) were added to Psyche J (1.8 mg, 1.5 µmol). The mixture was then stirred at room temperature for 12 hours, while shielding from light with an aluminum foil. Thereafter, the reaction solution diluted with water to 1 mL was purified by reverse phase HPLC (gradient: 20% for 5 min; 20-70% for 30 min, acetonitrile in a 50 mM triethylammonium acetate aqueous solution (pH 7.0), retention time = 28.9 min, YMC-Triart C18, flow rate = 10 mL/min) to obtain a target compound **8** (1.7 mg, 0.71 µmol, y. 56%, dark blue).
LRMS (ESI): *m*/*z* 1159.20 [M+2H]²⁺

### < Method of preparing HER2-recognizing protein >

Cupid molecules (SEQ ID NO: 1) (International Publication WO2015/125820) were fused with molecules recognizing the HER2 antigen (Löfblom, J. FEBS Lett. 584(12): 2670-80 (2010)) (SEQ ID NO: 2), and a protein having binding ability to both Psyche molecules (the above "Psyche J") and Her2/ErbB2 (hereafter this protein is referred to as "FL") was synthesized in *E. coli.* The gene sequence of FL (SEQ ID NO: 3) was synthesized as an artificial gene (SEQ ID NO: 4) by Eurofins. An expression vector was prepared using pET45b according to a common method. Specifically, using a primer set (Rv: catggtatatctccttcttaaagttaaac (SEQ ID NO: 5), Fw: cgcagcttaattaacctaggctgctgccac (SEQ ID NO: 6)), the vector was linearized by a PCR reaction. The sequence prepared by the artificial gene synthesis was amplified by a PCR reaction using a primer set (Fw: aggagatataccatgGTGGACAACAAATTCAACAAAGAG (SEQ ID NO: 7); Rv: gttaattaagctgcgTTAATGATGGTGGTGATGATGCGATG (SEQ ID NO: 8)). Bands were cut out of both of the PCR reaction products according to agarose gel electrophoresis, and were then purified. Using In-Fusion HD Cloning Kit (TaKaRa Bio), the purified vector and the insert were subjected to a ligation reaction and cloning in accordance with the dosage and administration described in the instruction manual.

A plasmid vector, which was confirmed by cloning and sequence analysis that a gene of interest had been incorporated therein, was introduced into competent cells BL21(DE3) (ECOS Competent *E. coli* BL21(DE3), Nippon Gene Co., Ltd.), so that the cells were transformed. A culture medium prepared by culturing the cells in 100 mL of 2 x YT medium overnight was inoculated into 1 L of a culture solution, and the obtained mixture was then cultured at 37°C. When the OD value at 600 nm became 0.5 to 0.8, IPTG was added to the culture to a final concentration of 0.5 mM, and the thus obtained mixture was then cultured at 37°C for 4 hours. Thereafter, the resulting cells were recovered by centrifugation (7500 x g, 20 min at 4°C).

The method of recovering IB from the cells is described below. After Benzonase (Merck) had been added to B-PER (Thermo Scientific) and had been suspended therein, the obtained mixture was incubated at room temperature for 10 minutes, and an insoluble fraction (inclusion body; hereinafter referred to as "IB") was separated from a soluble fraction by centrifugation. Thereafter, the IB is recovered. Subsequently, the recovered IB was resuspended in a 10-fold diluted B-PER buffer (without addition of Benzonase), and was centrifuged. The supernatant was discarded, and the washing of IB was repeated three times. After completion of the washing three times, the IB recovered by centrifugation was resuspended in ultrapure water (Milli Q). Thereafter, the resuspension was dispensed in an amount of 1 mL each into 1.5-mL tubes, and was then cryopreserved at -80°C.

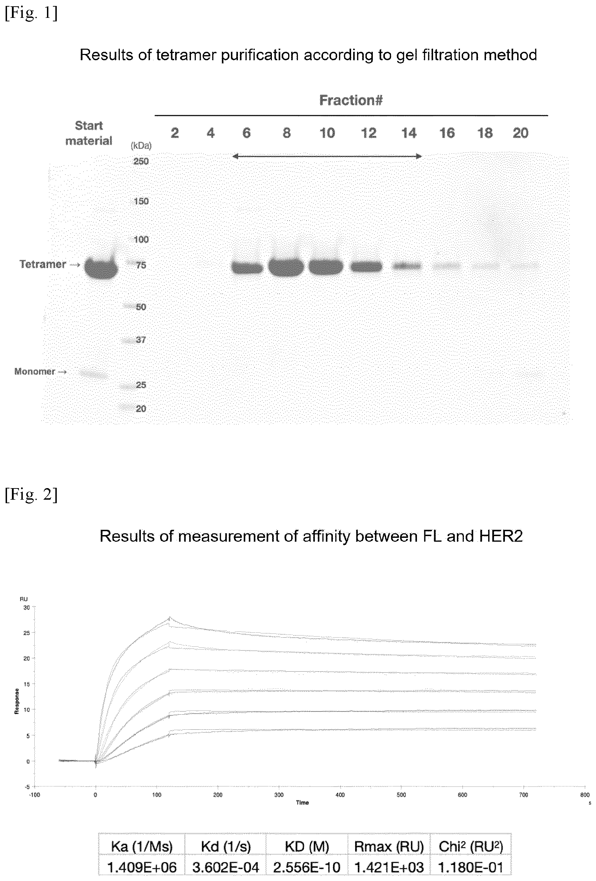

Denaturation and refolding of IB are described below. A denature buffer (6 M guanidium HCl, 200 mM NaCl, 50 mM Tris-HCl, and 1 mM EDTA; pH 8.0 at 4°C) was added to IB, and the IB was then dissolved in the buffer by pipetting. Thereafter, while stirring the obtained solution using a rotator, the solution was incubated at 4°C overnight, and was then centrifuged (15,000 x g, 20 min at 4°C) to recover a supernatant. The recovered supernatant was adjusted with a denature buffer, so the protein concentration (OD 280 nm) in the supernatant became 30 to 50 mg/mL. Thereafter, while stirring, the denatured protein solution having an adjusted concentration was added dropwise to a dilution buffer (PBS, or 200 mM NaCl, 50 mM Tris-HCl, and 1 mM EDTA; pH 8.0 at 4°C), in such an amount that the denatured protein solution was 500-fold diluted. Thereafter, the obtained solution was incubated at 4°C for 24 to 48 hours and was then purified with a Ni-NTA column (cOmplete, Merck). Subsequently, the resulting solution was concentrated using a centrifugal ultrafiltration filter to a concentration of 5 to 10 mg/mL, and the concentrated solution was then subjected to gel filtration purification with PBS, using a gel filtration purification column (HiLoad 16/60 Superdex 75 pg, GE Healthcare). The results of the purified fraction are shown in Fig. 1.

### < Performance evaluation of FL >

The purified FL was analyzed using SPR (Biacore T200, Cytiva), in terms of the binding activity to HER2 (ErbB2). The Her2 antigen (Recombinant Human ErbB2/Her2 Fc Chimera Protein, R & D SYSTEMS) was immobilized on a Sensor Chip CM5 (Cytiva) according to an amine coupling method (Amine Coupling Kit, Cytiva). Subsequently, the binding activity was measured using the FL dilution series (2-fold dilution series from 1E-08 M to 6.25E-10 M). The results are shown in Fig. 2. From the results, it was confirmed that FL exhibits stable binding activity to HER2.

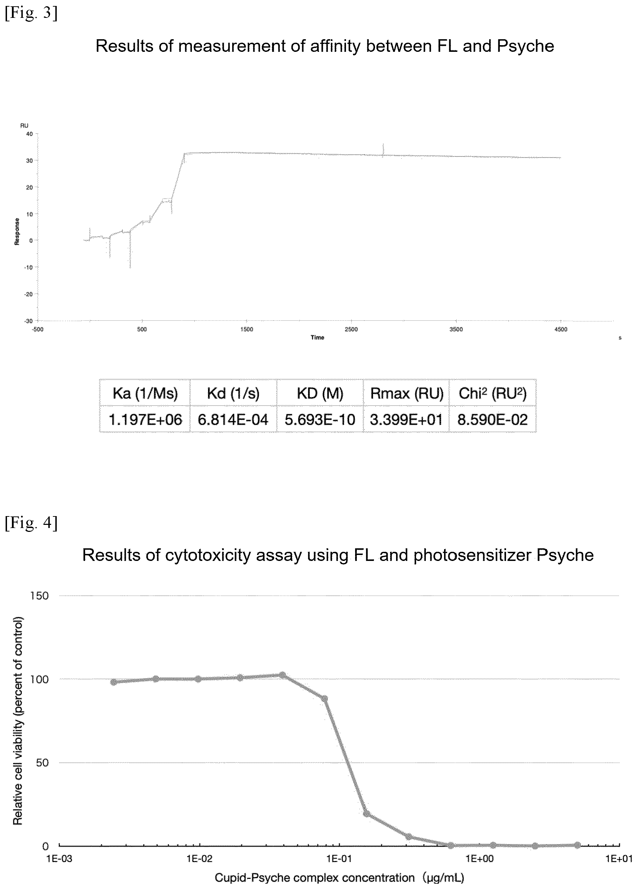

The purified FL was analyzed using SPR (Biacore T200, Cytiva), in terms of the binding activity to Psyche. The FL was immobilized on a Sensor Chip CM5 (Cytiva) according to the amine coupling method, and the binding activity of the FL to Psyche was then analyzed according to a single cycle kinetics method using Psyche dilution series (5 series, 2-fold dilution series from 1E-08 M to 6.25E-10 M) as analytes. The results are shown in Fig. 3. From the results, it was confirmed that FL exhibits stable binding activity to Psyche.

### < Cytotoxicity confirmed using FL and photosensitizer Psyche >.

Her2-positive cells (SK-BR-3, KPL-4) were seeded on a 96-well plate to result in a cell count of 1 × 10⁴ cells/well and a culture solution amount of 50 µL/well, and were then cultured overnight. FL and the photosensitizer Psyche (Compound 8 produced in the above Production Example 6) were mixed with each other to a molar ratio of 1 : 2 (hereinafter referred to as a "complex"), and 12 series of 2-fold dilution series were prepared from 10 µg/mL. After the dilution series had been prepared, the complex was added to the cells, and 24 hours later, the cells were irradiated with an LED emitting a 690-nm light at 100 J/cm² from the bottom surface of the culture plate. Thereafter, the cells were cultured for 24 hours, and a comparison was then made in terms of the number of living cells, using Cell Counting Kit-8 (DOJINDO LABORATORIES). A reagent was added in accordance with the dosage and administration described in the instruction manual, and the resulting cells were then incubated at 37°C in a CO₂ incubator for 1.5 hours. After that, the absorbance at 450 nm was measured, and the mean value was then calculated, followed by background correction. Thereafter, the control was set at 100%, and the cell proliferation percentage to the control under individual conditions was calculated. The results are shown in Fig. 4. It was confirmed that the complex of the FL and the photosensitizer Psyche compound has concentration-dependent cytotoxicity.

### < In vivo experiment >

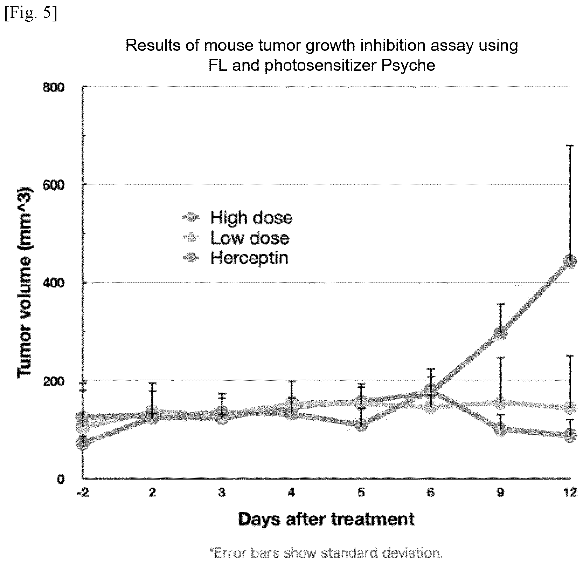

KPL-4 cells cultured in DMEM containing 10% FBS were transplanted into the subcutis or the second mammary gland of nude mice (5- to 10-week-old) in an amount of 5 x 10⁶ to 10 x 10⁶ cells/body. After completion of the transplantation, mice with a tumor mass volume of 50 to 200 mm³ were administered, via the caudal vein, with the complex of the FL and the photosensitizer Psyche at a dose of 75 to 150 µg/body. As a control drug, the mice were administered, via the caudal vein, with Herceptin (CHUGAI PHARMACEUTICAL CO., LTD.) at a dose of 150 µg/body. Five to six hours after the administration, the tumor mass portions of the mice administered with the complex of the FL and the photosensitizer Psyche were irradiated with an LED (USHIO) emitting a 690-nm light at 230 J/cm². After completion of the light irradiation, a body weight and a tumor mass volume were measured. The volume of the tumor mass was specifically obtained by measuring the long diameter and short diameter of the tumor mass with Vernier calipers, and then by applying the formula: (short diameter)² x long diameter x 1/2. The results are shown in Fig. 5. It was confirmed that the complex of the FL and the photosensitizer Psyche compound has a tumor proliferation-suppressing effect.

### < In vivo experiment 2 using FL and photosensitizer Psyche: Treatment of subcutaneously transplanted tumor >

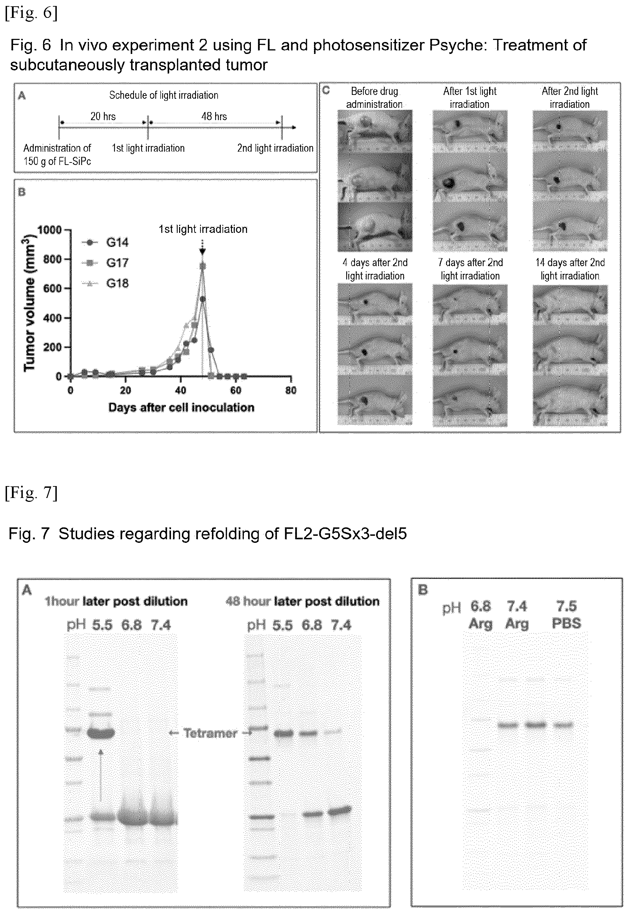

KPL-4 cells cultured in DMEM containing 10% FBS were transplanted into the subcutis of nude mice (5- to 10-week-old) in an amount of 5 x 10⁶ to 10 x 10⁶ cells/body. After completion of the transplantation, mice with a tumor mass volume of 500 to 800 mm³ were administered, via the caudal vein, with the complex of the FL and the photosensitizer Psyche at a dose of 150 µg/body. As shown in Fig. 6A, 20 hours and 68 hours after the drug administration, the tumor mass portions of the mice were irradiated with an LED (USHIO) emitting a 690-nm light at 230 J/cm² under anesthesia. After completion of the light irradiation, a body weight and a tumor mass volume were measured. The volume of the tumor mass was specifically obtained by measuring the long diameter and short diameter of the tumor mass with Vernier calipers, and then by applying the formula: (short diameter)² x long diameter x 1/2. As shown in Fig. 6B and Fig. 6C, regression of the tumor was observed immediately after the light irradiation, and after 14 weeks, the tumor disappeared, the skin wounds also disappeared, and thus, therapeutic effects were confirmed.

### < Construction of expression vector >

For the expression vector prepared in the above < Method of preparing HER2-recognizing protein >, an expression vector for producing FL2-G5Sx3-del5 having the amino acid sequence as set forth in SEQ ID NO: 10 formed by removing the 11 amino acids at the C-terminus, "PSAASHHHHHH," from the amino acid sequence as set forth in SEQ ID NO: 4 was produced using PCR according to site-specific mutagenesis for deleting a gene sequence of interest. Specifically, using a primer set (Fw: AAGTCAAATAACGCAGCTTAATTAACCTAG (SEQ ID NO: 11) and Rw: TGCGTTATTTGACTTTGGTAAAGGTGTCATG (SEQ ID NO: 12)), a PCR reaction was carried out using the expression vector as a template. Thereafter, the reaction solution was subjected to a DpnI treatment at 37°C for 1 hour, and *E. coli* was then transformed with the obtained reaction solution, followed by cloning. After completion of the cloning, a sequence analysis was carried out to confirm that the gene sequence as set forth in SEQ ID NO: 13 was incorporated. The vector was named "pET45-FL2-G5Sx3-del5."

Subsequently, protein expression vectors were constructed with a different length of an amino acid linker that links a molecule recognizing a HER2 antigen in FL2-G5Sx3-del5 to Cupid-del5. Specifically, a peptide formed by combining 4 glycine residues and 1 serine residue was set to be a basic unit, and expression vectors each having a linker with a length of 1 unit, 2 units or 3 units were produced. The production method thereof is as follows.

Using a primer set (Fw: GCGGAAGCCGGTATTACCGGGACCTGGTC (SEQ ID NO: 14); and Rw: TTTCGGAGCTTGAGCATCATTCAGTTTC (SEQ ID NO: 15)), pET45-FL2-G5Sx3-del5 was linearized. Complementary strand oligos were synthesized as follows, so that the oligos expressed amino acid linkers having each different length. The reaction was carried out at 95°C for 10 minutes, and thereafter, annealing was performed by natural cooling.

Oligo for a linker with 1 unit
   Fw: 
GAAACTGAATGATGCTCAAGCTCCGAAAGGAGGCGGAGGGTCTGCGGAAGCCG
GTATTACCGGGACCTGGTC (SEQ ID NO: 16)
   Rv: 
GACCAGGTCCCGGTAATACCGGCTTCCGCAGACCCTCCGCCTCCTTTCGGAGCTT
GAGCATCATTCAGTTTC (SEQ ID NO: 17)
Oligo for a linker with 2 units
   Fw: 
GCTCAAGCTCCGAAAGGAGGCGGAGGGTCTGGAGGTGGCGGTTCAGCGGAAGC
CGGTATT (SEQ ID NO: 18)
   Rv: 
AATACCGGCTTCCGCTGAACCGCCACCTCCAGACCCTCCGCCTCCTTTCGGAGCT
TGAGC (SEQ ID NO: 19)
Oligo for a linker with 3 units
   Fw: 
GCTCAAGCTCCGAAAGGAGGCGGAGGGTCTGGAGGTGGCGGTTCAGGTGGCGG
TGGCAGTGCGGAAGCCGGTATT (SEQ ID NO: 20)
   Rv:

AATACCGGCTTCCGCACTGCCACCGCCACCTGAACCGCCACCTCCAGACCCTCCG
CCTCCTTTCGGAGCTTGAGC (SEQ ID NO: 21)

Subsequently, using In-Fusion HD Cloning Kit (TaKaRa Bio), the purified vectors and the annealed oligos were subjected to a ligation reaction and cloning in accordance with the dosage and administration described in the instruction manual. Expression vectors, into which the gene sequences (SEQ ID NO: 25, SEQ ID NO: 26, and SEQ ID NO: 27) expressing the proteins with the respective linker lengths shown in SEQ ID NO: 22, SEQ ID NO: 23, and SEQ ID NO: 24, respectively, were confirmed to be incorporated, were named pET45-FL2-G4Sx1-del5, pET45-FL2-G4Sx2-del5, and pET45-FL2-G4Sx3-del5, respectively.

### < Studies regarding protein expression and purification method >

First of all, studies regarding expression and purification of FL2-G5Sx3-del5 were conducted. A plasmid vector, whose sequence was confirmed to be correct, was introduced into the competent cells BL21(DE3) (ECOS Competent *E. coli* BL21(DE3), Nippon Gene Co., Ltd.), so that the cells were transformed. A culture medium prepared by culturing the cells in 100 mL of 2 x YT medium overnight was inoculated into 1 L of a culture solution, and the obtained mixture was then cultured at 37°C. When the OD value at 600 nm became 0.5 to 0.8, IPTG was added to the culture to a final concentration of 0.5 mM, and the thus obtained mixture was then cultured at 37°C for 4 hours. Thereafter, the resulting cells were recovered by centrifugation (7500 x g, 20 min at 4°C).

The method of recovering IB from the cells is described below. After Benzonase (Merck) had been added to B-PER (Thermo Scientific) and had been suspended therein, the obtained mixture was incubated at room temperature for 10 minutes, and an insoluble fraction (inclusion body; hereinafter referred to as "IB") was separated from a soluble fraction by centrifugation. Thereafter, the IB is recovered. Subsequently, the recovered IB was resuspended in a 10-fold diluted B-PER buffer (without addition of Benzonase), and was centrifuged. The supernatant was discarded, and the washing of IB was repeated three times. After completion of the washing three times, the IB recovered by centrifugation was resuspended in ultrapure water (Milli Q). Thereafter, the resuspension was dispensed in an amount of 1 mL each into 1.5-mL tubes, and was then cryopreserved at -80°C.

Denaturation and refolding of IB are described below. A denature buffer (0.1 M Tris-HCl, pH 8.5, 6 M guanidine hydrochloride, and 10 mM EDTA) was added to IB, and the IB was then dissolved in the buffer by pipetting. Thereafter, while stirring the obtained solution using a rotator, the solution was incubated at 4°C overnight, and was then centrifuged (15,000 x g, 20 min at 4°C) to recover a supernatant. The recovered supernatant was adjusted with a denature buffer, so that the protein concentration (OD 280 nm) in the supernatant became 30 to 50 mg/mL. Thereafter, while stirring, the denatured protein solution having an adjusted concentration was added dropwise to a dilution buffer (PBS, or 200 mM NaCl, 50 mM Tris-HCl, and 1 mM EDTA; pH 8.0 at 4°C), in such an amount that the denatured protein solution was 500-fold diluted. Thereafter, the obtained solution was incubated at 4°C for 24 to 48 hours, and was then purified with a Ni-NTA column (cOmplete, Merck). Subsequently, the resulting solution was concentrated using a centrifugal ultrafiltration filter to a concentration of 5 to 10 mg/mL, and the concentrated solution was then subjected to gel filtration purification with PBS, using a gel filtration purification column (HiLoad 16/60 Superdex 75 pg, GE Healthcare).

### < Studies regarding refolding of FL2-G5Sx3-del5 >

An attempt was made to refold FL2-G5Sx3-del5, using the same refolding buffer as that described in "denaturation and refolding of IB" in the above < Method of preparing HER2-recognizing protein >. As a result, it was found that a large amount of FL2-G5Sx3-del5 was agglutinated upon dilution, and that a tetramer was hardly formed. In order to solve this problem, optimization of the tetramer formation by pH was progressed (Fig. 7). While stirring, the denatured protein solution having an adjusted concentration was added dropwise to a refolding buffer (50 mM sodium phosphate and 0.4 M arginine hydrochloride; pH 5.5), in such an amount that the denatured protein solution was 40- to 80-fold diluted. In this case, the formation of a tetramer was confirmed at an early stage (Fig. 7A). Further, it was confirmed that a majority of monomers disappeared as a result of incubation at 4°C for 48 hours. Moreover, it was also confirmed that FL-del5, which formed a tetramer at pH 5.5, then maintained the tetramer, although the buffer was replaced with a buffer at pH 6.8 to 7.5 (Fig. 7B).

Subsequently, centrifugation (12,000 x g, 20 min at 4°C) was carried out to remove insolubilized proteins. A supernatant was recovered and was then concentrated to 5 to 10 mg/mL, using a centrifugal ultrafiltration filter. Thereafter, the concentrate was subjected to gel filtration purification with a gel filtration buffer (0.1 M sodium phosphate and 0.2 M arginine hydrochloride; pH 6.8), using a gel filtration purification column (HiLoad 16/60 Superdex 75 pg, GE Healthcare).

### < Expression and refolding of FL2-G4Sx1-del5, FL2-G4Sx2-del5, and FL2-G4Sx3-del5 >

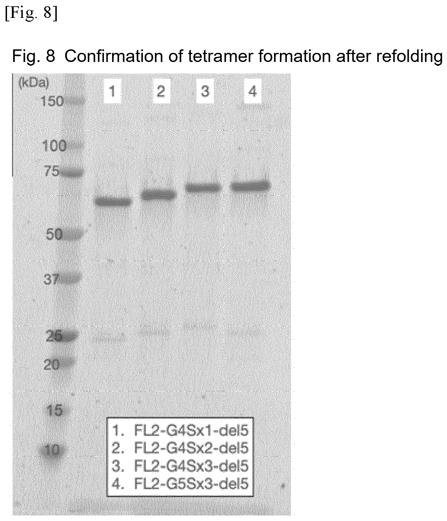

With regard to FL2-G4Sx1-del5, FL2-G4Sx2-del5, and FL2-G4Sx3-del5 as well, IB was prepared by the same method as that for FL2-G5Sx3-del5, and was then refolded using a refolding buffer at pH 5.5, so as to prepare a tetramer (Fig. 8).

### < Confirmation of cytotoxicity using FL2-G5Sx3-del5, FL2-G4Sx1-del5, FL2-G4Sx2-del5 and FL2-G4Sx3-del5, and photosensitizer Psyche >

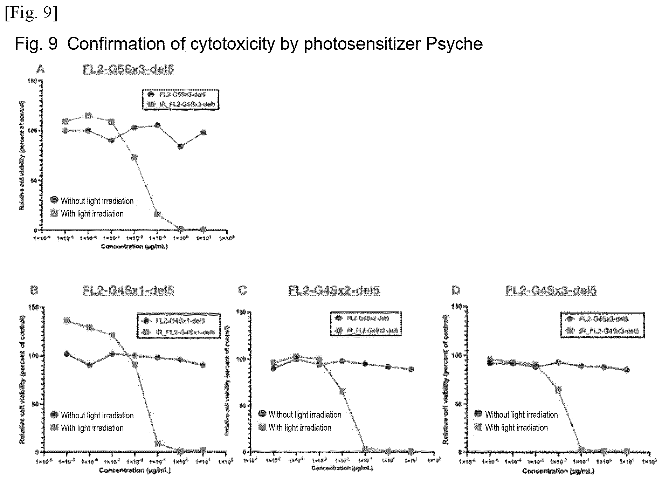

Her2-positive cells (KPL-4) were seeded on a 96-well plate to result in a cell count of 1 x 10⁴ cells/well and a culture solution amount of 50 µL/well, and were then cultured overnight. The purified FL2-G5Sx3-del5, FL2-G4Sx1-del5, FL2-G4Sx2-del5 or FL2-G4Sx3-del5 and the photosensitizer Psyche (Compound 8 produced in the above Production Example 6) were mixed with each other to a molar ratio of 1 : 2 (hereinafter referred to as a "complex"), and 8 series of 10-fold dilution series (including zero concentration) were prepared from 10 µg/mL. After the dilution series had been prepared, the complex was added to the cells, and 24 hours later, the cells were irradiated with an LED emitting a 690-nm light at 100 J/cm² from the bottom surface of the culture plate. Thereafter, the cells were cultured for 24 hours. After the complex-containing medium had been removed, the cells were washed with PBS once, and a fresh medium was then added. A comparison was then made in terms of the number of living cells, using Cell Counting Kit-8 (DOJINDO LABORATORIES). A reagent was added in accordance with the dosage and administration described in the instruction manual, and the resulting cells were then incubated at 37°C in a CO₂ incubator for 1.5 hours. After that, the absorbance at 450 nm was measured, and the mean value was then calculated, followed by background correction. Thereafter, the control was set at 100%, and the cell proliferation percentage to the control under individual conditions was calculated. The results are shown in (Figs. 9A to D). It was confirmed that each type of complex has light- and concentration-dependent cytotoxicity.

SEQ ID NO: 1 (Amino acid sequence of Cupid) 
AEAGITGTWSDQLGDTFIVTAGADGALTGTYENAVGGAESRYVLTGRYDSAPATDG
SGTALGWTVAWKNNSKNAHSATTWSGQYVGGADAKINTQWLLTSGTTNANAWKS
TLVGHDTFTKVKPSAASHHHHHH
SEQ ID NO: 2 (Amino acid sequence of HER2-recognizing protein) 
VDNKFNKEMRNAYWEIALLPNLNNQQKRAFIRSIYDDPSQSANLLAEAKKLNDAQ
APK
SEQ ID NO: 3 (FL artificial gene synthetic sequence) 
ATGGTGGACAACAAATTCAACAAAGAGATGCGCAATGCGTACTGGGAAATTGCC
CTGTTACCGAACCTGAACAACCAGCAGAAACGTGCCTTCATTCGCAGCATCTATG
ACGATCCAAGCCAATCTGCGAATCTGCTTGCAGAAGCGAAGAAACTGAATGATG
CTCAAGCTCCGAAAAGCGGAGGCGGCGGGTCTGGTGGCGGTGGCAGT
GGCGGAGGTGGCGGTTCAGCGGAAGCCGGTATTACCGGGACCTGGTCCGATCAA
CTCGGCGACACGTTTATCGTGACAGCAGGCGCGGATGGCGCGCTGACAGGGACG
TACGAGAATGCGGTAGGCGGTGCGGAAAGCCGCTATGTGTTGACCGGTCGTTAC
GATTCGGCTCCGGCTACGGATGGTTCCGGTACAGCCTTAGGGTGGACCGTTGCGT
 
GGAAGAACAACTCGAAGAATGCCCACAGTGCTACCACTTGGTCAGGCCAGTATG
TTGGCGGGGCGGATGCGAAAATCAACACTCAGTGGCTTCTGACCAGCGGAACCA
CGAATGCCAATGCGTGGAAATCCACGCTGGTCGGTCATGACACCTTTACCAAAGT
CAAACCGAGTGCAGCATCGCATCATCACCACCATCATTAA
SEQ ID NO: 4 (Amino acid sequence of FL) 
MVDNKFNKEMRNAYWEIALLPNLNNQQKRAFIRSIYDDPSQSANLLAEAKKLNDA
QAPKSGGGGSGGGGSGGGGSAEAGITGTWSDQLGDTFIVTAGADGALTGTYE
NAVGGAESRYVLTGRYDSAPATDGSGTALGWTVAWKNNSKNAHSATTWSGQYVGG
ADAKINTQWLLTSGTTNANAWKSTLVGHDTFTKVKPSAASHHHHHH
SEQ ID NO: 10 (Amino acid sequence of FL2-G5Sx3-del5) 
MVDNKFNKEMRNAYWEIALLPNLNNQQKRAFIRSIYDDPSQSANLLAEAKKLNDA
QAPKSGGGGSGGGGSGGGGSAEAGITGTWSDQLGDTFIVTAGADGALTGTYE
NAVGGAESRYVLTGRYDSAPATDGSGTALGWTVAWKNNSKNAHSATTWSGQYVGG
ADAKINTQWLLTSGTTNANAWKSTLVGHDTFTKVK
SEQ ID NO: 13 (Gene sequence of FL2-G5Sx3-del5) 
ATGGTGGACAACAAATTCAACAAAGAGATGCGCAATGCGTACTGGGAAATTGCC
CTGTTACCGAACCTGAACAACCAGCAGAAACGTGCCTTCATTCGCAGCATCTATG
ACGATCCAAGCCAATCTGCGAATCTGCTTGCAGAAGCGAAGAAACTGAATGATG
CTCAAGCTCCGAAAAGCGGAGGCGGCGGGTCTGGTGGCGGTGGCAGT
GGCGGAGGTGGCGGTTCAGCGGAAGCCGGTATTACCGGGACCTGGTCCGATCAA
CTCGGCGACACGTTTATCGTGACAGCAGGCGCGGATGGCGCGCTGACAGGGACG
TACGAGAATGCGGTAGGCGGTGCGGAAAGCCGCTATGTGTTGACCGGTCGTTAC
GATTCGGCTCCGGCTACGGATGGTTCCGGTACAGCCTTAGGGTGGACCGTTGCGT
GGAAGAACAACTCGAAGAATGCCCACAGTGCTACCACTTGGTCAGGCCAGTATG
TTGGCGGGGCGGATGCGAAAATCAACACTCAGTGGCTTCTGACCAGCGGAACCA
CGAATGCCAATGCGTGGAAATCCACGCTGGTCGGTCATGACACCTTTACCAAAGT
CAAATAA
SEQ ID NO: 22 (Amino acid sequence of FL2-G4Sx1-del5) 
MVDNKFNKEMRNAYWEIALLPNLNNQQKRAFIRSIYDDPSQSANLLAEAKKLNDA
QAPKGGGGSAEAGITGTWSDQLGDTFIVTAGADGALTGTYENAVGGAESRYVLTGR
 
YDSAPATDGSGTALGWTVAWKNNSKNAHSATTWSGQYVGGADAKINTQWLLTSGT
TNANAWKSTLVGHDTFTKVK
SEQ ID NO: 23 (Amino acid sequence of FL2-G4Sx2-del5) 
MVDNKFNKEMRNAYWEIALLPNLNNQQKRAFIRSIYDDPSQSANLLAEAKKLNDA
QAPKGGGGSGGGGSAEAGITGTWSDQLGDTFIVTAGADGALTGTYENAVGGAESR
YVLTGRYDSAPATDGSGTALGWTVAWKNNSKNAHSATTWSGQYVGGADAKINTQ
WLLTSGTTNANAWKSTLVGHDTFTKVK
SEQ ID NO: 24 (Amino acid sequence of FL2-G4Sx3-del5) 
MVDNKFNKEMRNAYWEIALLPNLNNQQKRAFIRSIYDDPSQSANLLAEAKKLNDA
QAPKGGGGSGGGGSGGGGSAEAGITGTWSDQLGDTFIVTAGADGALTGTYENAVG
GAESRYVLTGRYDSAPATDGSGTALGWTVAWKNNSKNAHSATTWSGQYVGGADA
KINTQWLLTSGTTNANAWKSTLVGHDTFTKVK
SEQ ID NO: 25 (Gene sequence of FL2-G4Sx1-del5) 
ATGGTGGACAACAAATTCAACAAAGAGATGCGCAATGCGTACTGGGAAATTGCC
CTGTTACCGAACCTGAACAACCAGCAGAAACGTGCCTTCATTCGCAGCATCTATG
ACGATCCAAGCCAATCTGCGAATCTGCTTGCAGAAGCGAAGAAACTGAATGATG
CTCAAGCTCCGAAAGGAGGCGGAGGGTCTGCGGAAGCCGGTATTACCGGGACCT
GGTCCGATCAACTCGGCGACACGTTTATCGTGACAGCAGGCGCGGATGGCGCGC
TGACAGGGACGTACGAGAATGCGGTAGGCGGTGCGGAAAGCCGCTATGTGTTGA
CCGGTCGTTACGATTCGGCTCCGGCTACGGATGGTTCCGGTACAGCCTTAGGGTG
GACCGTTGCGTGGAAGAACAACTCGAAGAATGCCCACAGTGCTACCACTTGGTC
AGGCCAGTATGTTGGCGGGGCGGATGCGAAAATCAACACTCAGTGGCTTCTGAC
CAGCGGAACCACGAATGCCAATGCGTGGAAATCCACGCTGGTCGGTCATGACAC
CTTTACCAAAGTCAAATAA
SEQ ID NO: 26 (Gene sequence of FL2-G4Sx2-del5) 
ATGGTGGACAACAAATTCAACAAAGAGATGCGCAATGCGTACTGGGAAATTGCC
CTGTTACCGAACCTGAACAACCAGCAGAAACGTGCCTTCATTCGCAGCATCTATG
ACGATCCAAGCCAATCTGCGAATCTGCTTGCAGAAGCGAAGAAACTGAATGATG
CTCAAGCTCCGAAAGGAGGCGGAGGGTCTGGAGGTGGCGGTTCAGCGGAAGCC
GGTATTACCGGGACCTGGTCCGATCAACTCGGCGACACGTTTATCGTGACAGCAG
 
GCGCGGATGGCGCGCTGACAGGGACGTACGAGAATGCGGTAGGCGGTGCGGAA
AGCCGCTATGTGTTGACCGGTCGTTACGATTCGGCTCCGGCTACGGATGGTTCCG
GTACAGCCTTAGGGTGGACCGTTGCGTGGAAGAACAACTCGAAGAATGCCCACA
GTGCTACCACTTGGTCAGGCCAGTATGTTGGCGGGGCGGATGCGAAAATCAACA
CTCAGTGGCTTCTGACCAGCGGAACCACGAATGCCAATGCGTGGAAATCCACGC
TGGTCGGTCATGACACCTTTACCAAAGTCAAATAA
SEQ ID NO: 27 (Gene sequence of FL2-G4Sx3-del5)

ATGGTGGACAACAAATTCAACAAAGAGATGCGCAATGCGTACTGGGAAATTGCC
CTGTTACCGAACCTGAACAACCAGCAGAAACGTGCCTTCATTCGCAGCATCTATG
ACGATCCAAGCCAATCTGCGAATCTGCTTGCAGAAGCGAAGAAACTGAATGATG
CTCAAGCTCCGAAAGGAGGCGGAGGGTCTGGAGGTGGCGGTTCAGGTGGCGGT
GGCAGTGCGGAAGCCGGTATTACCGGGACCTGGTCCGATCAACTCGGCGACACG
TTTATCGTGACAGCAGGCGCGGATGGCGCGCTGACAGGGACGTACGAGAATGCG
GTAGGCGGTGCGGAAAGCCGCTATGTGTTGACCGGTCGTTACGATTCGGCTCCGG
CTACGGATGGTTCCGGTACAGCCTTAGGGTGGACCGTTGCGTGGAAGAACAACT
CGAAGAATGCCCACAGTGCTACCACTTGGTCAGGCCAGTATGTTGGCGGGGCGG
ATGCGAAAATCAACACTCAGTGGCTTCTGACCAGCGGAACCACGAATGCCAATG
CGTGGAAATCCACGCTGGTCGGTCATGACACCTTTACCAAAGTCAAATAA

### In vivo experiment

### (1) Materials and methods

### < Reagents, etc. >

The *Escherichia coli* strain BL21(DE3) (Catalog No.: 312-06534, Nippon Gene, Tokyo, Japan) was used as an expression host. The pET-45b(+) vector was used in cloning and gene expression analysis (Catalog No.: 71327; Novagen, Madison, WI, USA). The denature buffer consisted of 0.1 M Tris-HCl, pH 8.5, 10 mM EDTA, and 6 M guanidine hydrochloride. The refolding buffer consisted of 0.1 M sodium phosphate and 0.4 M arginine-HCl, pH 6.0. The gel filtration buffer consisted of 0.1 M sodium phosphate and 0.2 M arginine-HCl, pH 6.5.

The photosensitizer Psyche-Ax-SiPC was Compound 8 produced in the above Production Example 6 (Yamatsugu K, Katoh H, Yamashita T, et al., Antibody mimetic drug conjugate manufactured by high-yield Escherichia coli expression and non-covalent binding system. Protein Expr Purif. 2022; 192: 106043. doi:10.1016/j.pep.2021.106043; and Takahashi K, Sugiyama A, Ohkubo K, et al., Axially substituted silicon phthalocyanine payloads for antibody-drug conjugates. Synlett. 2021; 32: 1098-1103. doi:10.1055/a-1503-6425).

Human breast cancer KPL-4 cells were provided by Prof. Kurebayashi (Kawasaki Medical School, Kurashiki, Japan). Kadcyla (trastuzumab emtansine) was purchased from Roche (Basel, Switzerland).

### < Preparation of recombinant Z_{HER2:342}-Cupid-His >

The recombinant Z_{HER2:342}-Cupid-His protein was the same as that described in the above < Method of preparing HER2-recognizing protein >. This recombinant protein was produced from an *E. coli* inclusion body according to denaturation using a denaturing agent and a direct dilution refolding method. The expression and purification of the recombinant Z_{HER2:342}-Cupid-His protein have been described in detail, so far (Sugiyama A, Kawamura T, Tanaka T, et al., Cupid and Psyche system for the diagnosis and treatment of advanced cancer. Proc Jpn Acad Ser B Phys Biol Sci. 2019; 95: 602-611. doi:10.2183/pjab.95.041). Briefly describing, the *E. coli* strain BL21(DE3) was transformed with the expression vector pET45b(+)-Z_{HER2:342}-Cupid-His, and the recombinant Z_{HER2:342}-Cupid-His protein was allowed to express therein as an inclusion body (IB). The purified IB was solubilized in a denature buffer. The solubilized IB solution was clarified by centrifugation (12,000 x g, at 4°C for 15 minutes), and was then refolded by directly applying 40-fold dilution with a refolding buffer. After incubation at 4°C for 72 hours, the refolded tetrameric recombinant Z_{HER2:342}-Cupid-His protein was purified with a gel filtration buffer, using a gel filtration column (HiLoad 16/600 Superdex 75pg, #28989333, Cytiva, Marlborough, MA, USA). The purity of the tetrameric recombinant Z_{HER2:342}-Cupid-His protein was analyzed according to non-reducing (no boiling and no reducing agent) SDS-PAGE (TGX Stain-Free FastCast 12%, #1610185, Bio-Rad, Hercules, CA, USA). The binding activity of the tetrameric recombinant Z_{HER2:342}-Cupid-His protein to HER2 and Psyche-Ax-SiPC was analyzed according to surface plasmon resonance using Biacore T200 (Cytiva, Marlborough, MA, USA).

### < In vivo breast cancer tumor model experiment >

### < Preparation of animal models >

The KPL-4 cell line was maintained in DMEM (low glucose) (FUJIFILM Wako Pure Chemical Corporation, Osaka, Japan) supplemented with 10% FBS, 100 U/mL penicillin, and 100 µg/mL streptomycin (#15140122, Thermo Fisher Scientific, Waltham, MA, USA) (Kurebayashi J, Otsuki T, Tang CK, et al. Isolation and characterization of a new human breast cancer cell line, KPL-4, expressing the Erb B family receptors and interleukin-6. Br J Cancer. 1999; 79: 707-717. doi:10.1038/sj.bjc.6690114). The KPL-4 cells (7,500,000) were subcutaneously transplanted into the thigh of BALB/cSlc-nu/nu nude mice (Sankyo Lab Service, Inc., Tokyo, Japan). Proliferation of the subcutaneous tumor was monitored by measuring a tumor volume (0.5 x length x width²) using Vernier calipers, and the body weight of the animals was then monitored as an indicator of treatment-related toxicity. The tumor size of 20 mice increased at 44 days after the transplantation, and reached approximately 400 mm³. These 20 mice were randomly classified into two groups each consisting of 10 mice, namely, a Kadcyla (registered trademark) group and a Z_{HER2:342}-Cupid-His-Ax-SiPC group.

### < In vivo experimental design >

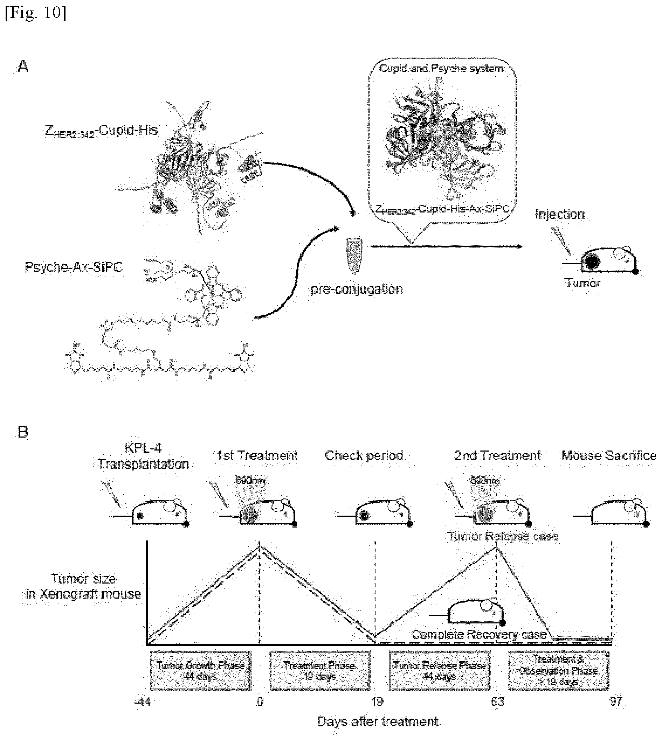

Kadcyla (registered trademark) and Z_{HER2:342}-Cupid-His-Ax-SiPC (Fig. 10A) were each injected into xenograft mouse models on Day 0. In this study, in order to evaluate tumor recurrence, the observation period was set to be approximately 19 days after the initial treatment. In the case of tumor recurrence, a second treatment was carried out at 44 days after the confirmation period of the recurrence of cancer cell proliferation (at 63 days after the first treatment) that is the same period before the first treatment. Finally, the xenograft mouse models were sacrificed at 97 days after the treatment, namely, at 19 days or more after the second treatment. Fig. 10B shows an outline view of the experimental design.

### < Preparation of therapeutic drug >

Kadcyla (registered trademark) was prepared according to the manufacturer's instructions. Preparation of Z_{HER2:342}-Cupid-His and Psyche-Ax-SiPC was as previously reported (Yamatsugu K, Katoh H, Yamashita T, et al., Antibody mimetic drug conjugate manufactured by high-yield Protein Expr Purif. 2022; 192: 106043. doi:10.1016/j.pep.2021.106043). Briefly speaking, Psyche-Ax-SiPC solubilized in dimethyl sulfoxide at a concentration of 5 mM was preserved as a stock solution at -80°C. In order to prepare a complex, Z_{HER2:342}-Cupid-His and Psyche-Ax-SiPC were mixed with each other at a molar ratio of 1 : 2 in a dark place on ice for 10 minutes. A concentrate of Z_{HER2:342}-Cupid-His-Ax-SiPC was diluted using a phosphate-buffered saline.

### < Treatment of mouse models >

Kadcyla (registered trademark) and Z_{HER2:342}-Cupid-His-Ax-SiPC were administered to the mice at doses of 300 µg and 150 µg. Twenty hours after the injection, the mice in the Z_{HER2:342}-Cupid-His-Ax-SiPC group were irradiated with a 690-nm light emitting diode light (Yamato Scientific Co., Ltd., Tokyo, Japan) at 230 J/cm⁻². Forty-eight hours after the initial light irradiation, the tumor was irradiated again in the same manner as that described above.

### < Pathological analysis >

Skin samples from xenograft tumor sites, and samples from other important organs including lung, heart, kidney, liver and gastrointestinal tract, were obtained from the mice. These samples were fixed in a 4% paraformaldehyde phosphate buffer (#163-20145, FUJIFII,M Wako Pure Chemical Corporation, Osaka, Japan) at 4°C for 24 hours, and were then embedded in paraffin according to standard histopathological procedures. For H & E staining, histopathological test pieces were deparaffinized by being immersed in xylene (#241-00091, FUJIFII,M Wako Pure Chemical Corporation, Osaka, Japan) at room temperature for 10 minutes, and were then rehydrated by being immersed in ethanol (#057-00451, FUJIFII,M Wako Pure Chemical Corporation, Osaka, Japan). A hematoxylin (#6187-4P, Sakura Finetek Japan, Tokyo, Japan) and eosin (#8660, Sakura Finetek Japan, Tokyo, Japan) solution was used for the H & E staining according to the manufacturer's protocols. Stained slides were dehydrated again by being immersed in ethanol and then being immersed in xylene. Glass coverslips containing Marinol (#4197193; Muto Pure Chemicals, Tokyo, Japan) were used to cover the stained slides. The H & E-stained slides were histologically examined using the OLYMPUS cellSens Standard system (OLYMPUS, Tokyo, Japan).

### < Statistical analysis >

A p value of less than 0.05 (p < 0.05) was set to be statistically significant. All graphs, calculations, and statistical analyses were performed using the statistical package for Microsoft Excel (Microsoft Corp., Redmond, WA, USA).

### (2) Results

### (2-1) Local AMDC treatment (treatment with Z_{HER2:342}-Cupid-His-Ax-SiPC) rapidly reduced the tumor volume in xenograft mouse models, but led to recurrence in some cases.

In order to establish mouse xenograft models with HER2-positive breast cancer, KPL-4 cells were subcutaneously transplanted into BALB/cSlc-nu/nu nude mice. The tumor volume in the 20 mice increased after the KPL-4 cell injection, reaching an average volume of 400±110 mm³ (125 to 551 mm³) at 44 days after the cell transplantation. The KPL-4 xenograft mouse models were randomly divided into two treatment groups (n = 10 per group). Kadcyla (registered trademark) was injected into the mice in Group 1 on Day 0 (mean ± SD: 417 ± 88), whereas Z_{HER2:342}-Cupid-His-Ax-SiPC was injected into the mice in Group 2 on Day 0 (mean ± SD: 384 ± 126). Both groups were irradiated on Day 1 (with an interval of 24 hours after the injection) and on Day 3 (with an interval of 72 hours after the injection. There was no statistically significant difference in terms of the tumor volume in the mice between the Kadcyla (registered trademark) group and the Z_{HER2:342}-Cupid-His-Ax-SiPC group.

In order to evaluate the effects of the local AMDC treatment, changes over time in the tumor volumes in the KPL-4 xenograft mouse models after the treatment with Kadcyla (registered trademark) and Z_{HER2:342}-Cupid-His-Ax-SiPC were measured. Fig. 11 shows changes in the tumor volumes in the KPL-4 xenograft mouse models after the injection with Kadcyla (registered trademark) and Z_{HER2:342}-Cupid-His-Ax-SiPC. In both groups, a reduction in the tumor size was observed after the first treatment. There were significant differences in the tumor volume reduction between the Kadcyla (registered trademark) group and the Z_{HER2:342}-Cupid-His-Ax-SiPC group on the 4th day (p <0.001), the 7th day (p <0.001), the 11th day (p <0.001), the 14th day (p <0.01), and the 17th day (p <0.05) after the treatment (Fig. 11A).

The tumor volume in the KPL-4 xenograft mouse models was gradually decreased after the treatment with Kadcyla (registered trademark), and in only 1 case, the tumor volume was decreased to 0 mm³ at 21 days after the injection (Figs. 11B and D), while the tumor size in the same xenograft models was rapidly decreased after the treatment with Z_{HER2:342}-Cupid-His-Ax-SiPC. Furthermore, about half of the tumors in the mice disappeared at 11 days after the injection with Z_{HER2:342}-Cupid-His-Ax-SiPC (Figs. 11C and D). The mean tumor volume was smallest around 20 days after the treatment and during the recurrence check period. However, after the observation period, the tumor volume in the xenograft mouse models was persisted and then, was gradually increased again in 5 out of the 10 cases. In some cases, the tumor size became larger in the Z_{HER2:342}-Cupid-His-Ax-SiPC group at 2 months after the injection (the mean volume on the 63rd day: 381±296 mm³). In contrast, a steady decrease in the tumor size was observed in the Kadcyla (registered trademark) group.

These data show that a short-term local AMDC treatment has strong antitumor effects against HER2-positive breast cancer, but may lead to a risk of tumor recurrence after the initial treatment in some cases.

### (2-2) The recurrent tumor was rapidly reduced by the second local AMDC treatment, and then, it completely disappeared.

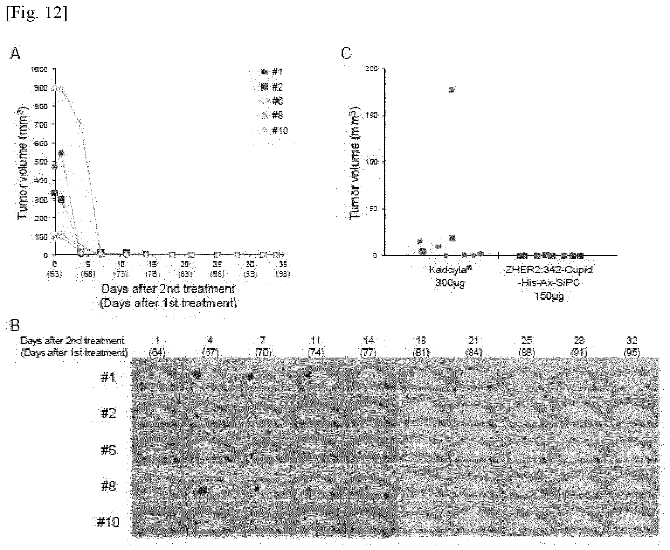

In order to examine the efficacy of repeated local AMDC treatments, a Z_{HER2:342}-Cupid-His-Ax-SiPC pre-conjugate was injected into the xenograft mouse models at 63 days after the first treatment (at 44 days after the tumor observation period), and the size of the recurrent tumor was then measured. Fig. 12 shows changes in the sizes of 5 recurrent tumors after the second Z_{HER2:342}-Cupid-His-Ax-SiPC treatment. The tumor volume in the xenograft mouse models was decreased more rapidly after the second Z_{HER2:342}-Cupid-His-Ax-SiPC treatment than after the first Z_{HER2:342}-Cupid-His-Ax-SiPC treatment. Furthermore, the tumors disappeared in all mice at 7 days after the second Z_{HER2:342}-Cupid-His-Ax-SiPC injection (Figs. 12A and B), and no reproliferation was observed even though more than one month passed after the tumor recurrence occurring after the second Z_{HER2:342}-Cupid-His-Ax-SiPC treatment. There was a significant difference in the tumor size between 0 day and 97 days after the first Z_{HER2:342}-Cupid-His-Ax-SiPC treatment. Furthermore, the size of a tumor mass in the Z_{HER2:342}-Cupid-His-Ax-SiPC repeated treatment group was smaller than that in the Kadcyla (registered trademark) treatment group at 97 days after the first treatment (Fig. 12C). These results suggest that repeated local AMDC treatments rapidly and completely dissipated the recurrent tumors.

### (2-3) Local AMDC treatment exhibited robust effects against HER2-positive xenograft tumor models without causing skin degeneration.

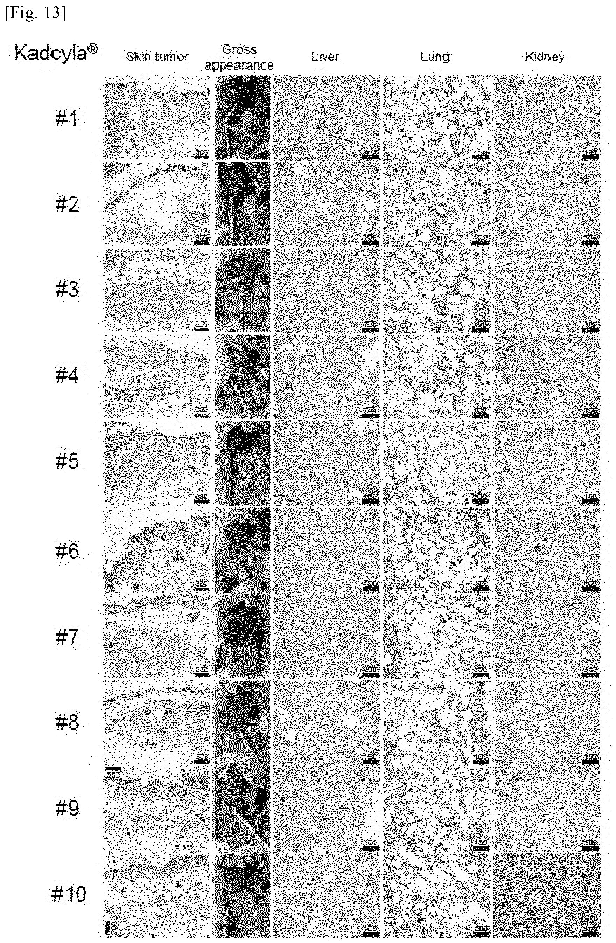

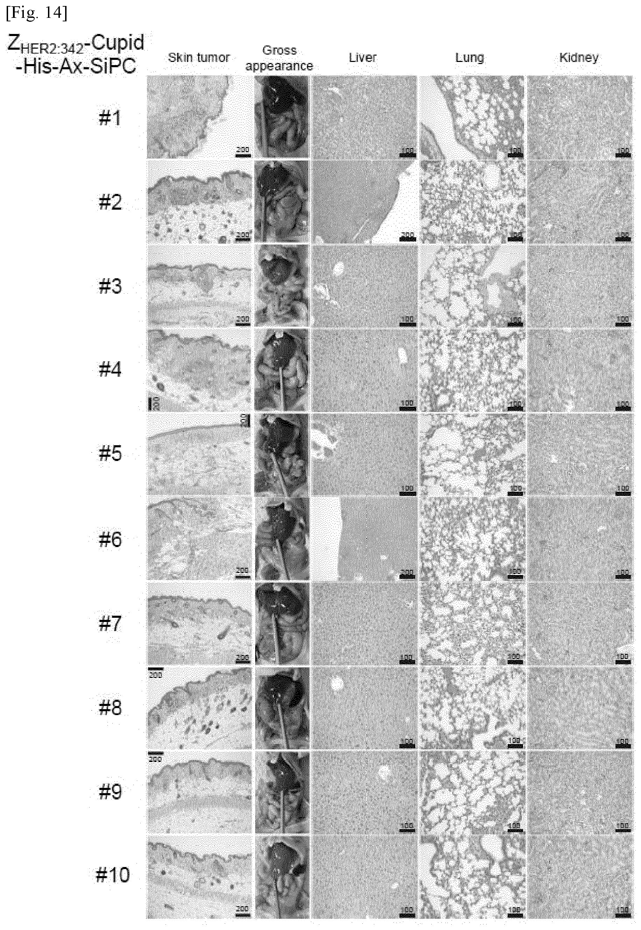

In order to evaluate the pathological response to the local AMDC treatment, histological examination was performed on the KPL-4 xenograft mouse models on the 97th day after the Kadcyla (registered trademark) and Z_{HER2:342}-Cupid-His-Ax-SiPC treatments. Histologically, no residual tumor cells were observed in the subcutaneous region around the xenograft KPL-4 tumor site on the 97th day, in either the mice (n = 10) treated with Kadcyla (registered trademark) or the mice (n = 10) treated by the second AMDC treatment (Fig. 13 and Fig. 14). No metastatic tumors were observed in lymph nodes or distant organs in both of the two types of mice (Fig. 13 and Fig. 14). Therefore, it was concluded that pathological complete remission was achieved in both of the two groups. According to further histological observation, a granulomatous reaction with a nidus of hemosiderin-containing macrophages and/or local calcification were frequently observed in the subcutaneous region of the Kadcyla (registered trademark)-treated mice (6/10 [mice #1, 2, 3, 7, 8 and 10], 60%) (Fig. 13). In one (mouse #2) of the Kadcyla (registered trademark)-treated mice, subcutaneous cysts surrounded by granuloma tissues were also observed. In contrast, tumor granulomatous reactions were rarely observed in the second AMDC-treated group (1/10 [mouse #6], 10%) (Fig. 14) (p <0.001, chi-square test). The frequency of granulomatous reactions in the Kadcyla (registered trademark) treatment group suggested that the Kadcyla (registered trademark) treatment be likely to induce a higher level of tissue damage attended with hemorrhagic and necrotic changes, rather than the AMDC treatment. After the second local AMDC treatment, pathological complete remission of xenograft KPL-4 tumors was clearly achieved. Furthermore, no histologically apparent side effects were observed in vital organs, except for microscopically localized hepatic necrosis observed in two mice (mice #2 and #6) (Fig. 14).

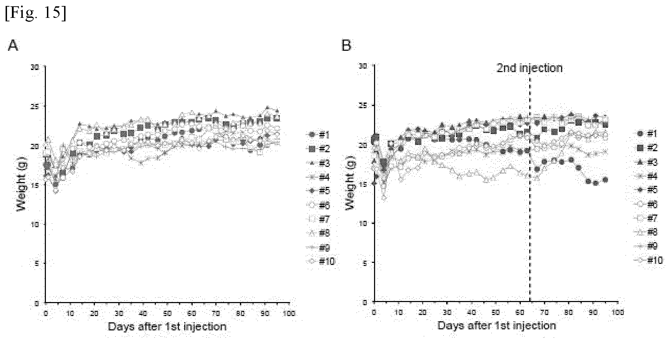

(2-4) Changes over time in the body weights of xenografts were measured after the Kadcyla (registered trademark) and Z_{HER2:342}-Cupid-His-Ax-SiPC treatments. The body weights of the xenograft mouse models after the Kadcyla (registered trademark) treatment showed a moderate tendency of increasing the body weight in all individual mice (Fig. 15A). One of the xenograft Z_{HER2:342}-Cupid-His-Ax-SiPC-treated mice in the tumor recurrence cases showed a reduction in the body weight from 3 weeks after the first treatment, but after the second treatment, the body weight was sharply increased (Fig. 15B). Another xenograft Z_{HER2:342}-Cupid-His-Ax-SiPC-treated mouse in the recurrence cases showed a reduction in the body weight even after the second treatment, but this weight reduction may be caused by unrelated infection that was incidentally discovered during the histopathological examination. These results demonstrate that local AMDC has strong therapeutic effects against xenograft mouse models and has fewer side effects than the Kadcyla (registered trademark) treatment.

## Claims

1. A fusion protein, wherein an antigen-binding molecule having a molecular weight of 20,000 or less is bound, via a linker sequence, to the N-terminal side and/or C-terminal side of the amino acid sequence as set forth in SEQ ID NO: 1, provided that the C-terminal amino acid sequence consisting of Pro-Ser-Ala-Ala-Ser His-His-His-His-His-His may be partially or entirely deleted.

2. The fusion protein according to claim 1, which has the antigen-binding molecule having a molecular weight of 20,000 or less, the linker sequence, and the amino acid sequence as set forth in SEQ ID NO: 1, provided that the C-terminal amino acid sequence consisting of Pro-Ser-Ala-Ala-Ser His-His-His-His-His-His may be partially or entirely deleted, in this order from the N-terminal side to the C-terminal side.

3. The fusion protein according to claim 1 or 2, wherein the antigen-binding molecule is a molecule that binds to an antigen expressing in a cancer cell.

4. The fusion protein according to any one of claims 1 to 3, wherein the antigen-binding molecule is a molecule that binds to Her2.

5. The fusion protein according to any one of claims 1 to 4, wherein the antigen-binding molecule has the amino acid sequence as set forth in SEQ ID NO: 2.

6. The fusion protein according to any one of claims 1 to 5, wherein the linker sequence consists of a glycine residue(s) and a serine residue(s) and the number of the amino acid residues is 5 to 25.

7. The fusion protein according to any one of claims 1 to 6, wherein the linker sequence is an amino acid sequence represented by [(Gly)ₘ-Ser]ₙ wherein m represents an integer of 1 to 10, and n represents an integer of 1 to 5.

8. The fusion protein according to any one of claims 1 to 7, which has the amino acid sequence as set forth in SEQ ID NO: 4, provided that the C-terminal amino acid sequence consisting of Pro-Ser-Ala-Ala-Ser His-His-His-His-His-His may be partially or entirely deleted.

9. A nucleic acid encoding the fusion protein having the amino acid sequence as set forth in SEQ ID NO: 4, provided that the C-terminal amino acid sequence consisting of Pro-Ser-Ala-Ala-Ser His-His-His-His-His-His may be partially or entirely deleted.

10. A cancer therapeutic agent or a cancer diagnostic agent, comprising the fusion protein according to any one of claims 1 to 8.

11. A kit for treating or diagnosing cancer, comprising (1) the fusion protein according to any one of claims 1 to 8, and (2) a conjugate of a compound represented by the following formula (1) or a salt thereof, and a diagnostic substance or a therapeutic substance: 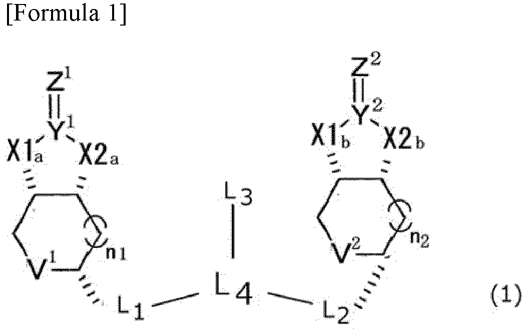 wherein
X1a, X1b, X2a and X2b each independently represent O or NH,
Y¹ and Y² each independently represent C or S,
Z¹ and Z² each independently represent O, S or NH,
V¹ and V² each independently represent S or S⁺-O⁻, n1 and n2 each independently represent an integer of 0 or 1,
L₁ and L₂ each independently represent a divalent linking group,
L₃ represents a group comprising a functional group capable of binding to the diagnostic substance or the therapeutic substance at the terminus thereof, and
L₄ represents a trivalent linking group.

12. The kit according to claim 11, wherein the diagnostic substance or the therapeutic substance is a phthalocyanine dye.

13. A method for producing the fusion protein according to any one of claims 1 to 8, comprising a step of allowing a nucleic acid encoding the fusion protein according to any one of claims 1 to 8 to express in a host.

14. The method according to claim 13, wherein the fusion protein is allowed to express in a bacterial inclusion body and is then recovered.
